(19) Europäisches Patentamt
European Patent Office
Office européen des brevets

(11) EP 0 894 857 A2

(12) **EUROPEAN PATENT APPLICATION**

(43) Date of publication:
**03.02.1999 Bulletin 1999/05**

(21) Application number: **98305872.8**

(22) Date of filing: **23.07.1998**

(51) Int Cl.6: **C12N 15/31**, C07K 14/315, C07K 16/12, C12Q 1/68, A61K 38/16

(84) Designated Contracting States:
**AT BE CH CY DE DK ES FI FR GB GR IE IT LI LU MC NL PT SE**
Designated Extension States:
**AL LT LV MK RO SI**

(30) Priority: **01.08.1997 US 54568 P**
**13.03.1998 US 999720**

(71) Applicants:
- **SMITHKLINE BEECHAM CORPORATION**
**Philadelphia Pennsylvania 19103 (US)**
- **SMITHKLINE BEECHAM PLC**
**Brentford, Middlesex TW8 9EP (GB)**

(72) Inventors:
- **Shilling, Lisa Kathleen**
**King of Prussia, Pennsylvania 19406 (US)**
- **Traini, Christopher Michael**
**King of Prussia, Pennsylvania 19406 (US)**
- **Jaworski, Deborah Dee**
**King of Prussia, Pennsylvania 19406 (US)**
- **Wang, Min**
**King of Prussia, Pennsylvania 19406 (US)**
- **Wilding, Edwina Imogen**
**Brentford, Middlesex TW8 9EP (GB)**
- **O'Dwyer, Karen Mary**
**Brentford, Middlesex TW8 9EP (GB)**

(74) Representative:
**Connell, Anthony Christopher et al**
**SmithKline Beecham plc**
**Corporate Intellectual Property,**
**Two New Horizons Court**
**Brentford, Middlesex TW8 9EP (GB)**

(54) **SecA gene from Streptococcus pneumoniae**

(57) The invention provides SecA polypeptides and polynucleotides encoding SecA polypeptides and methods for producing such polypeptides by recombinant techniques. Also provided are methods for utilizing SecA polypeptides to screen for antibacterial compounds.

EP 0 894 857 A2

## Description

### RELATED APPLICATIONS

This application claims benefit of US Provisional Patent Application Serial No. 60/054,568 filed August 1, 1997.

### FIELD OF THE INVENTION

This invention relates to newly identified polynucleotides and polypeptides, and their production and uses, as well as their variants, agonists and antagonists, and their uses. In particular, the invention relates to polynucleotides and polypeptides of the SecA family, hereinafter referred to as "SecA".

### BACKGROUND OF THE INVENTION

The Streptococci make up a medically important genera of microbes known to cause several types of disease in humans, including, for example, otitis media, conjunctivitis, pneumonia, bacteremia, meningitis, sinusitis, pleural empyema and endocarditis, and most particularly meningitis, such as for example infection of cerebrospinal fluid. Since its isolation more than 100 years ago, Streptococcus pneumoniae has been one of the more intensively studied microbes. For example, much of our early understanding that DNA is, in fact, the genetic material was predicated on the work of Griffith and of Avery, Macleod and McCarty using this microbe. Despite the vast amount of research with S. pneumoniae, many questions concerning the virulence of this microbe remain. It is particularly preferred to employ Streptococcal genes and gene products as targets for the development of antibiotics.

The frequency of *Streptococcus pneumoniae* infections has risen dramatically in the past few decades. This has been attributed to the emergence of multiply antibiotic resistant strains and an increasing population of people with weakened immune systems. It is no longer uncommon to isolate *Streptococcus pneumoniae* strains which are resistant to some or all of the standard antibiotics. This phenomenon has created a demand for both new anti-microbial agents, vaccines, and diagnostic tests for this organism.

Clearly, there exists a need for factors, such as the SecA embodiments of the invention, that have a present benefit of being useful to screen compounds for antibiotic activity. Such factors are also useful to determine their role in pathogenesis of infection, dysfunction and disease. There is also a need for identification and characterization of such factors and their antagonists and agonists to find ways to prevent, ameliorate or correct such infection, dysfunction and disease.

Certain of the polypeptides of the invention possess amino acid sequence homology to a known secA protein. secA gene sequences available in the public domain include: Listeria monocytogenes, Gen Bank accession number L32090 Staphylococcus carnosus, Gen Bank accession number X79725 Klein, M., Meens, J. and Freudl, R. (1995). Functional characterization of the Staphylococcus carnosus SecA protein in Escherichia coli and Bacillus subtilis secA mutant strains. FEMS Microbiol. Lett. 131 (3), 271-277. Bacillus subtilis Gen Bank accession number D10279; Sadaie, Y., Takamatsu, H., Nakamura, K. and Yamane, K. (1991). Sequencing reveals similarity of the wild-type div+ gene of Bacillus subtilis to the Escherichia coli secA gene. Gene 98 (1), 101-105.; Staphylococcus aureus GenBank accession number U97062.

### SUMMARY OF THE INVENTION

It is an object of the invention to provide polypeptides that have been identified as SecA polypeptides by homology between the amino acid sequence set out in Table I [SEQ ID NO: 2 or 4] and a known amino acid sequence or sequences of other proteins such as secA protein.

It is a further object of the invention to provide polynucleotides that encode SecA polypeptides, particularly polynucleotides that encode the polypeptide herein designated SecA.

In a particularly preferred embodiment of the invention the polynucleotide comprises a region encoding SecA polypeptides comprising a sequence set out in Table 1 [SEQ ID NO: 1 or 3] which includes a full length gene, or a variant thereof.

In another particularly preferred embodiment of the invention there is a SecA protein from *Streptococcus pneumoniae* comprising the amino acid sequence of Table 1 [SEQ ID NO:2 or 4], or a variant thereof.

As a further aspect of the invention there are provided isolated nucleic acid molecules encoding SecA, particularly *Streptococcus pneumoniae* SecA, including mRNAs, cDNAs, genomic DNAs. Further embodiments of the invention include biologically, diagnostically, prophylactically, clinically or therapeutically useful variants thereof, and compositions comprising the same.

In accordance with another aspect of the invention, there is provided the use of a polynucleotide of the invention for therapeutic or prophylactic purposes, in particular genetic immunization. Among the particularly preferred embodiments of the invention are naturally occurring allelic variants of SecA and polypeptides encoded thereby.

In another aspect of the invention there are provided polypeptides of *Streptococcus pneumoniae* referred to herein as SecA as well as biologically, diagnostically, prophylactically, clinically or therapeutically useful variants thereof, and compositions comprising the same.

Among the particularly preferred embodiments of the invention are variants of SecA polypeptide encoded by naturally occurring alleles of the SecA gene.

In a preferred embodiment of the invention there are provided methods for producing the aforementioned SecA polypeptides.

In accordance with yet another aspect of the invention, there are provided inhibitors to such polypeptides, useful as antibacterial agents, including, for example, antibodies.

In accordance with certain preferred embodiments of the invention, there are provided products, compositions and methods for assessing SecA expression, treating disease, assaying genetic variation, and administering a SecA polypeptide or polynucleotide to an organism to raise an immunological response against a bacteria, especially a *Streptococcus pneumoniae* bacteria.

In accordance with certain preferred embodiments of this and other aspects of the invention there are provided polynucleotides that hybridize to SecA polynucleotide sequences, particularly under stringent conditions.

In certain preferred embodiments of the invention there are provided antibodies against SecA polypeptides.

In other embodiments of the invention there are provided methods for identifying compounds which bind to or otherwise interact with and inhibit or activate an activity of a polypeptide or polynucleotide of the invention comprising: contacting a polypeptide or polynucleotide of the invention with a compound to be screened under conditions to permit binding to or other interaction between the compound and the polypeptide or polynucleotide to assess the binding to or other interaction with the compound, such binding or interaction being associated with a second component capable of providing a detectable signal in response to the binding or interaction of the polypeptide or polynucleotide with the compound; and determining whether the compound binds to or otherwise interacts with and activates or inhibits an activity of the polypeptide or polynucleotide by detecting the presence or absence of a signal generated from the binding or interaction of the compound with the polypeptide or polynucleotide.

In accordance with yet another aspect of the invention, there are provided SecA agonists and antagonists, preferably bacteriostatic or bacteriocidal agonists and antagonists.

In a further aspect of the invention there are provided compositions comprising a SecA polynucleotide or a SecA polypeptide for administration to a cell or to a multicellular organism.

In another embodiment of the invention there is provided a computer readable medium having stored thereon a member selected from the group consisting of: a polynucleotide comprising the sequence of SEQ ID NO. 1 or 3; a polypeptide comprising the sequence of SEQ ID NO. 2 or 4; a set of polynucleotide sequences wherein at least one of said sequences comprises the sequence of SEQ ID NO. 1 or 3; a set of polypeptide sequences wherein at least one of said sequences comprises the sequence of SEQ ID NO. 2 or 4; a data set representing a polynucleotide sequence comprising the sequence of SEQ ID NO.1 or 3; a data set representing a polynucleotide sequence encoding a polypeptide sequence comprising the sequence of SEQ ID NO. 2 or 4; a polynucleotide comprising the sequence of SEQ ID NO. 1; a polypeptide comprising the sequence of SEQ ID NO. 2; a set of polynucleotide sequences wherein at least one of said sequences comprises the sequence of SEQ ID NO. 1; a set of polypeptide sequences wherein at least one of said sequences comprises the sequence of SEQ ID NO. 2; a data set representing a polynucleotide sequence comprising the sequence of SEQ ID NO. 1; a data set representing a polynucleotide sequence encoding a polypeptide sequence comprising the sequence of SEQ ID NO. 2. A further embodiment of the invention provides a computer based method for performing homology identification, said method comprising the steps of providing a polynucleotide sequence comprising the sequence of SEQ ID NO. 1 in a computer readable medium; and comparing said polynucleotide sequence to at least one polynucleotide or polypeptide sequence to identify homology.

A further embodiment of the invention provides a computer based method for performing homology identification, said method comprising the steps of: providing a polypeptide sequence comprising the sequence of SEQ ID NO. 2 in a computer readable medium; and comparing said polypeptide sequence to at least one polynucleotide or polypeptide sequence to identify homology.

A further embodiment of the invention provides a computer based method for polynucleotide assembly, said method comprising the steps of: providing a first polynucleotide sequence comprising the sequence of SEQ ID NO. 1 in a computer readable medium; and screening for at least one overlapping region between said first polynucleotide sequence and a second polynucleotide sequence.

A further embodiment of the invention provides a computer based method for performing homology identification, said method comprising the steps of: providing a polynucleotide sequence comprising the sequence of SEQ ID NO. 1 in a computer readable medium; and comparing said polynucleotide sequence to at least one polynucleotide or polypeptide sequence to identify homology.

A further embodiment of the invention provides a computer based method for performing homology identification, said method comprising the steps of: providing a polypeptide sequence comprising the sequence of SEQ ID NO. 2 in a computer readable medium; and comparing said polypeptide sequence to at least one polynucleotide or polypeptide sequence to identify homology.

A further embodiment of the invention provides a computer based method for polynucleotide assembly, said method comprising the steps of: providing a first polynucleotide sequence comprising the sequence of SEQ ID NO. 1 in a computer readable medium; and screening for at least one overlapping region between said first polynucleotide sequence and a second polynucleotide sequence.

Various changes and modifications within the spirit and scope of the disclosed invention will become readily apparent to those skilled in the art from reading the following descriptions and from reading the other parts of the present disclosure.

## DESCRIPTION OF THE INVENTION

The invention relates to SecA polypeptides and polynucleotides as described in greater detail below. In particular, the invention relates to polypeptides and polynucleotides of a SecA of *Streptococcus pneumoniae,* which is related by amino acid sequence homology to secA polypeptide. The invention relates especially to SecA having the nucleotide and amino acid sequences set out in Table 1 as SEQ ID NO: 1 and SEQ ID NO: 2 respectively.

## TABLE 1

### SecA Polynucleotide and Polypeptide Sequences

(A) Sequences from *Streptococcus pneumoniae* SecA polynucleotide sequence [SEQ ID NO:1].

5'-
ATGGCTAATATTTTAAAAACAATTATCGAAAATGATAAAGGAGAAATCCGTCGTCTGGAAAAGATGGCTG
ACAAGGTTTTCAAATACGAAGACCAAATGGCTGCTTTGACTGACGACCAACTAAAAGCAAAAACAGTTGA
ATTTAAGGAACGTTATCAAAATGGAGAATCACTGGATTCATTGCTTTACGAAGCATTTGCGGTTGTCCGT
GAAGGTGCCAAACGTGTCCTAGGTCTCTTCCCTTATAAGGTTCAGGTCATGGGGGGATTGTTCTTCACC
ATGGTGACGTGCCAGAGATGCGTACAGGGGAAGGAAAACCTTGACTGCGACCATGCCGGTATACCTCAA
TGCCCTTTCAGGTAAAGGGGTTCACGTAGTTACGGTTAATGAATACCTGTCAGAACGTGACGCGACTGAG
ATGGGTGAATTGTACTCTTGGCTTGGTTTGTCAGTAGGGATTAACTTGGCTACCAAATCTCCAATGGAGA
AAAAAGAAGCCTATGAGTGTGATATTACTTACTCAACTAACTCAGAAATCGGATTTGACTACCTTCGTGA
CAATATGGTCGTTCGCGCTGAAAACATGGTACAACGTCCGCTTAACTATGCCTTGGTCGATGAGGTTGAC
TCTATCTTGATTGACGAGGCTCGTACACCTTTGATTGTATCAGGTGCCAATGCGGTTGAAACCAGTCAGT
TGTATCACATGGCAGACCACTATGTAAAATCTTTGAACAAAGATGACTACATCATCGATGTGCAGTCTAA
GACTATTGGTTTGTCTGATTCAGGGATTGACAGGGCTGAAAGCTACTTCAAACTTGAAAACCTCTATGAC
ATCGAAAACGTGGCTTTGACCCACTTTATCGATAACGCCCTTCGTGCCAACTACATCATGCTTCTCGATA
TTGACTATGTGGTGAGCGAAGAGCAAGAAATCTTGATTGTCGACCAATTTACAGGTCGTACCATGGAAGG
TCGTCGTTATTCTGATGGATTGCACCAAGCTATTGAAGCCAAAGAAGGTGTGCCAATCCAGGATGAAACC
AAGACATCTGCCTCAATCACGTACCAAAACCTTTTCCGTATGTACAAAAAATTGTCTGGTATGACGGGTA
CAGGTAAGACTGAGGAAGAAGAATTTCGTGAAATCTACAACATTCGTGTTATTCCAATCCCAACAAACCG
TCCTGTTCAACGTATTGACCACTCAGACCTTCTTTATGCAAGTATCGAATCTAAGTTTAAAGCGGTTGTC
GAAGACGTTAAGGCTCGTTACCAAAAGGGTCAACCTGTCTTGGTTGGTACAGTAGCGGTTGAAACTAGTG
ACTACATTTCTAAGAAATTGGTTGCAGCTGGTGTTCCTCACGAAGTCTTGAATGCCAAAAACCACTATAG
AGAAGCCCAAATCATCATGAATGCTGGTCAACGTGGTGCCGTTACCATCGCAACCAACATGGCGGGTCGT
GGTACCGACATCAAGCTTGGTGAAGGTGTTCGTGAACTTGGAGGACTTTGTGTTATTGGTACAGAACGTC
ATGAAAGTCGTCGTATCGATAACCAGCTTCGTGGACGTTCAGGTCGTCAAGGAGATCCAGGTGAGTCACA
ATTCTACCTATCTCTTGAAGATGATTTGATGAAACGTTTTGGTTCTGAACGCTTGAAGGGAATCTTTGAA
CGCTTGAACATGTCTGAAGAGGCCATTGAGTCTCGCATGTTGACGCGTCAGGTTGAAGCAGCTCAGAAAC
GTGTCGAAGGAAATAACTACGATACCCGTAAACAAGTCCTTCAATACGATGATGTCATGCGTGAACAACG
TGAGATTATCTATGCTCAACGTTACGATGTCATCACTGCAGATCGTGACTTGGCACCTGAAATTCAGTCT
ATGATTAAGCGCACGATTGAACGTGTCGTTGATGGTCATGCGCGTGCCAAACAAGATGAAAAACTAGAGG

CAATTTTGAACTTTGCTAAGTACAACTTGCTTCCTGAAGATTCTATTACGATGGAAGACTTGTCAGGCTT
GTCTGATAAGGCCATCAAGGAAGAGCTTTTCCAACGTGCCTTGAAGGTTTACGATAGTCAGGTTTCAAAA
CTACGCGATGAAGAAGCAGTTAAAGAATTCCAAAAAGTTTTGATTCTACGAGTGGTGGATAACAAGTGGA
CAGATCATATCGATGCCCTTGATCAATTGCGTAACGCGGTTGGACTTCGTGGCTATGCTCAGAACAACCC
TGTTGTTGAGTATCAGGCAGAAGGTTTCCGTATGTTTAATGATATGATTGGTTCGATTGAGTTTGATGTG
ACACGCTTGATGATGAAAGCACAAATTCATGAACAAGAAAGACCACAGGCAGAACGTCATATCAGTACAA
CAGCGACTCGCAATATCGCTGCTCACCAAGCAAGTATGCTAGAAGATTTGGATTTGAGCCAGATTGGACG
CAATGAACTTTGCCCATGTGGTTCTGGTAAGAAATTTAAAAACTGTCACGGTAAAAGACAA-3'

(B) *Streptococcus pneumoniae* SecA polypeptide sequence deduced from the polynucleotide sequence in this table [SEQ ID NO:2].

$NH_2$-
MANILKTIIENDKGEIRRLEKMADKVFKYEDQMAALTDDQLKAKTVEFKERYQNGESLDSLLYEAFAVVR
EGAKRVLGLFPYKVQVMGGIVLHHGDVPEMRTGEGKTLTATMPVYLNALSGKGVHVVTVNEYLSERDATE
MGELYSWLGLSVGINLATKSPMEKKEAYECDITYSTNSEIGFDYLRDNMVVRAENMVQRPLNYALVDEVD
SILIDEARTPLIVSGANAVETSQLYHMADHYVKSLNKDDYIIDVQSKTIGLSDSGIDRAESYFKLENLYD
IENVALTHFIDNALRANYIMLLDIDYVVSEEQEILIVDQFTGRTMEGRRYSDGLHQAIEAKEGVPIQDET
KTSASITYQNLFRMYKKLSGMTGTGKTEEEEFREIYNIRVIPIPTNRPVQRIDHSDLLYASIESKFKAVV
EDVKARYQKGQPVLVGTVAVETSDYISKKLVAAGVPHEVLNAKNHYREAQIIMNAGQRGAVTIATNMAGR
GTDIKLGEGVRELGGLCVIGTERHESRRIDNQLRGRSGRQGDPGESQFYLSLEDDLMKRFGSERLKGIFE
RLNMSEEAIESRMLTRQVEAAQKRVEGNNYDTRKQVLQYDDVMREQREIIYAQRYDVITADRDLAPEIQS
MIKRTIERVVDGHARAKQDEKLEAILNFAKYNLLPEDSITMEDLSGLSDKAIKEELFQRALKVYDSQVSK
LRDEEAVKEFQKVLILRVVDNKWTDHIDALDQLRNAVGLRGYAQNNPVVEYQAEGFRMFNDMIGSIEFDV
TRLMMKAQIHEQERPQAERHISTTATRNIAAHQASMLEDLDLSQIGRNELCPCGSGKKFKNCHGKRQ-
COOH

(C) Polynucleotide sequences comprising *Streptococcus pneumoniae* SecA ORF sequence [SEQ ID NO:3].

5'-
TACTCTTGGCTTGGTTTGTCAGTAGGGATTAACTTGGCTACCAAATCTCCAATGGAGAAAAAAGAAGCCT
ATGAGTGTGATATTACTTACTCAACTAACTCAGAAATCGGATTTGACTACCTTCGTGACAATATGGTCGT
TCGCGCTGAAAACATGGTACAACGTCCGCTTAACTATGCCTTGGTCGATGAGGTTGACTCTATCTTGATT
GACGAGGCTCGTACACCTTTGATTGTATCAGGTGCCAATGCGGTTGAAACCAGTCAGTTGTATCACATGG
CAGACCACTATGTAAAATCTTTGAACAAAGATGACTACATCATCGATGTGCAGTCTAAGACTATTGGTTT
GTCTGATTCAGGGATTGACAGGGCTGAAAGCTACTTCAAACTTGAAAACCTCTATGACATCGAAAACGTG
GCTTTGACCCACTTTATCGATAACGCCCTTCGTGCCAACTACATCATGCTTCTCGATATTGACTATGTGG
TGAGCGAAGAGCAAGAAATCTTGATTGTCGACCAATTTACAGGTCGTACCATGGAAGGTCGTCGTTATTC
TGATGGATTGCACCAAGCTATTGAAGCCAAAGAAGGTGTGCCAATCCAGGATGAAACCAAGACATCTGCC
TCAATCACGTACCAAAACCTTTTCCGTATGTACAAAAAATTGTCTGGTATGACGGGTACAGGTAAGACTG

AGGAAGAAGAATTTCGTGAAATCTACAACATTCGTGTTATTCCAATCCCAACAAACCGTCCTGTTCAACG
TATTGACCACTCAGACCTTCTTTATGCAAGTATCGAATCTAAGTTTAAAGCGGTTGTCGAAGACGTTAAG
GCTCGTTACCAAAAGGGTCAACCTGTCTTGGTTGGTACAGTAGCGGTTGAAACTAGTGACTACATTTCTA
AGAAATTGGTTGCAGCTGGTGTTCCTCACGAAGTCTTGAATGCCAAAAACCACTATAGAGAAGCCCAAAT
CATCATGAATGCTGGTCAACGTGGTGCCGTTACCATCGCAACCAACATGGCGGGTCGTGGTACCGACATC
AAGCTTGGTGAAGGTGTTCGTGAACTTGGAGGACTTTGTGTTATTGGTACAGAACGTCATGAAAGTCGTC
GTATCGATAACCAGCTTCGTGGACGTTCAGGTCGTCAAGGAGATCCAGGTGAGTCACAATTCTACCTATC
TCTTGAAGATGATTTGATGAAACGTTTTGGTTCTGAACGCTTGAAGGGAATCTTTGAACGCTTGAACATG
TCTGAAGAGGCCATTGAGTCTCGCATGTTGACGCGTCAGGTTGAAGCAGCTCAGAAACGTGTCGAAGGAA
ATAACTACGATACCCGTAAACAAGTCCTTCAATACGATGATGTCATGCGTGAACAACGTGAGATTATCTA
TGCTCAACGTTACGATGTCATCACTGCAGATCGTGACTTGGCACCTGAAATTCAGTCTATGATTAAGCGC
ACGATTGAACGTGTCGTTGATGGTCATGCGCGTGCCAAACAAGATGAAAAACTAGAGGCAATTTTGAACT
TTGCTAAGTACAACTTGCTTCCTGAAGATTCTATTACGATGGAAGACTTGTCAGGCTTGTCTGATAAGGC
CATCAAGGAAGAGCTTTTCCAACGTGCCTTGAAGGTTTACGATAGTCAGGTTTCAAAACTACGCGATGAA
GAAGCAGTTAAAGAATTCCAAAAAGTTTTGATTCTACGAGTGGTGGATAACAAGTGGACAGATCATATCG
ATGCCCTTGATCAATTGCGTAACGCGGTTGGACTTCGTGGCTATGCTCAGAACAACCCTGTTGTTGAGTA
TCAGGCAGAAGGTTTCCGTATGTTTAATGATATGATTGGTTCGATTGAGTTTGATGTGACACGCTTGATG
ATGAAAGCACAAATTCATGAACAAGAAAGACCACAGGCAGAACGTCATATCAGTACAACAGCGACTCGCA
ATATCGCTGCTCACCAAGCAAGTATGCTAGAAGATTTGGATTTGAGCCAGATTGGACGCAATGAACTTTG
CCCATGTGGTTCTGGTAAGAAATTTAAAAACTGTCACGGTAAAAGACAA-3'

(D) *Streptococcus pneumoniae* SecA polypeptide sequence deduced from the polynucleotide ORF sequence in this table [SEQ ID NO:4].

$NH_2$-
YSWLGLSVGINLATKSPMEKKEAYECDITYSTNSEIGFDYLRDNMVVRAENMVQRPLNYALVDEVDSILI
DEARTPLIVSGANAVETSQLYHMADHYVKSLNKDDYIIDVQSKTIGLSDSGIDRAESYFKLENLYDIENV
ALTHFIDNALRANYIMLLDIDYVVSEEQEILIVDQFTGRTMEGRRYSDGLHQAIEAKEGVPIQDETKTSA
SITYQNLFRMYKKLSGMTGTGKTEEEEFREIYNIRVIPIPTNRPVQRIDHSDLLYASIESKFKAVVEDVK
ARYQKGQPVLVGTVAVETSDYISKKLVAAGVPHEVLNAKNHYREAQIIMNAGQRGAVTIATNMAGRGTDI
KLGEGVRELGGLCVIGTERHESRRIDNQLRGRSGRQGDPGESQFYLSLEDDLMKRFGSERLKGIFERLNM
SEEAIESRMLTRQVEAAQKRVEGNNYDTRKQVLQYDDVMREQREIIYAQRYDVITADRDLAPEIQSMIKR
TIERVVDGHARAKQDEKLEAILNFAKYNLLPEDSITMEDLSGLSDKAIKEELFQRALKVYDSQVSKLRDE
EAVKEFQKVLILRVVDNKWTDHIDALDQLRNAVGLRGYAQNNPVVEYQAEGFRMFNDMIGSIEFDVTRLM
MKAQIHEQERPQAERHISTTATRNIAAHQASMLEDLDLSQIGRNELCPCGSGKKFKNCHGKRQ-COOH

---

**Deposited materials**

A deposit containing a *Streptococcus pneumoniae* 0100993 strain has been deposited with the National Collections of Industrial and Marine Bacteria Ltd. (herein "NCIMB"), 23 St. Machar Drive, Aberdeen AB2 1RY, Scotland on 11 April 1996 and assigned deposit number 40794. The deposit was described as Streptococcus pneumoniae 0100993 on deposit. On 17 April 1996 a Streptococcus pneumoniae 0100993 DNA library in E. coli was similarly deposited with the NCIMB and assigned deposit number 40800. The *Streptococcus pneumoniae* strain deposit is referred to herein as "the deposited strain" or as "the DNA of the deposited strain."

The deposited strain contains the full length SecA gene. The sequence of the polynucleotides contained in the deposited strain, as well as the amino acid sequence of the polypeptide encoded thereby, are controlling in the event of any conflict with any description of sequences herein.

The deposit of the deposited strain has been made under the terms of the Budapest Treaty on the International Recognition of the Deposit of Micro-organisms for Purposes of Patent Procedure. The strain will be irrevocably and without restriction or condition released to the public upon the issuance of a patent. The deposited strain is provided merely as convenience to those of skill in the art and is not an admission that a deposit is required for enablement, such as that required under 35 U.S.C. §112.

A license may be required to make, use or sell the deposited strain, and compounds derived therefrom, and no such license is hereby granted.

One aspect of the invention there is provided an isolated nucleic acid molecule encoding a mature polypeptide expressible by the *Streptococcus pneumoniae* 0100993 strain contained in the deposited strain. Further provided by the invention are SecA nucleotide sequences of the DNA in the deposited strain and amino acid sequences encoded thereby. Also provided by the invention are SecA polypeptide sequences isolated from the deposited strain and amino acid sequences derived therefrom.

**Polypeptides**

The polypeptides of the invention include a polypeptide of Table 1 [SEQ ID NO:2 or 4] (in particular the mature polypeptide) as well as polypeptides and fragments, particularly those which have the biological activity of SecA, and also those which have at least 70% identity to a polypeptide of Table 1 [SEQ ID NO: 1 or 3]or the relevant portion, preferably at least 80% identity to a polypeptide of Table 1 [SEQ ID NO:2 or 4and more preferably at least 90% identity to a polypeptide of Table 1 [SEQ ID NO:2 or 4] and still more preferably at least 95% identity to a polypeptide of Table 1 [SEQ ID NO:2 or 4] and also include portions of such polypeptides with such portion of the polypeptide generally containing at least 30 amino acids and more preferably at least 50 amino acids.

The invention also includes polypeptides of the formula:

$$X\text{-}(R_1)_m\text{-}(R_2)\text{-}(R_3)_n\text{-}Y$$

wherein, at the amino terminus, X is hydrogen or a metal, and at the carboxyl terminus, Y is hydrogen or a metal, $R_1$ and $R_3$ are any amino acid residue, m is an integer between 1 and 1000 or zero, n is an integer between 1 and 1000 or zero, and $R_2$ is an amino acid sequence of the invention, particularly an amino acid sequence selected from Table 1. In the formula above $R_2$ is oriented so that its amino terminal residue is at the left, bound to $R_{1,}$ and its carboxy terminal residue is at the right, bound to $R_3$. Any stretch of amino acid residues denoted by either R group, where m and/or n is greater than 1, may be either a heteropolymer or a homopolymer, preferably a heteropolymer.

A fragment is a variant polypeptide having an amino acid sequence that entirely is the same as part but not all of the amino acid sequence of the aforementioned polypeptides. As with SecA polypeptides fragments may be "free-standing," or comprised within a larger polypeptide of which they form a part or region, most preferably as a single continuous region, a single larger polypeptide.

Preferred fragments include, for example, truncation polypeptides having a portion of an amino acid sequence of Table 1 [SEQ ID NO:2 or 4], or of variants thereof, such as a continuous series of residues that includes the amino terminus, or a continuous series of residues that includes the carboxyl terminus. Degradation forms of the polypeptides of the invention in a host cell, particularly a *Streptococcus pneumoniae,* are also preferred. Further preferred are fragments characterized by structural or functional attributes such as fragments that comprise alpha-helix and alpha-helix forming regions, beta-sheet and beta-sheet-forming regions, turn and turn-forming regions, coil and coil-forming regions, hydrophilic regions, hydrophobic regions, alpha amphipathic regions, beta amphipathic regions, flexible regions, surface-forming regions, substrate binding region, and high antigenic index regions.

Also preferred are biologically active fragments which are those fragments that mediate activities of SecA, including those with a similar activity or an improved activity, or with a decreased undesirable activity. Also included are those fragments that are antigenic or immunogenic in an animal, especially in a human. Particularly preferred are fragments comprising receptors or domains of enzymes that confer a function essential for viability of *Streptococcus pneumoniae* or the ability to initiate, or maintain cause disease in an individual, particularly a human.

Variants that are fragments of the polypeptides of the invention may be employed for producing the corresponding full-length polypeptide by peptide synthesis; therefore, these variants may be employed as intermediates for producing the full-length polypeptides of the invention.

In addition to the standard single and triple letter representations for amino acids, the term "X" or "Xaa" may also be used in describing certain polypeptides of the invention. "X" and "Xaa" mean that any of the twenty naturally occurring amino acids may appear at such a designated position in the polypeptide sequence.

**Polynucleotides**

Another aspect of the invention relates to isolated polynucleotides, including the full length gene, that encode the SecA polypeptide having a deduced amino acid sequence of Table 1 [SEQ ID NO:2 or 4] and polynucleotides closely related thereto and variants thereof.

Using the information provided herein, such as a polynucleotide sequence set out in Table 1 [SEQ ID NO: 1 or 3], a polynucleotide of the invention encoding SecA polypeptide may be obtained using standard cloning and screening methods, such as those for cloning and sequencing chromosomal DNA fragments from bacteria using *Streptococcus pneumoniae* 0100993 cells as starting material, followed by obtaining a full length clone. For example, to obtain a polynucleotide sequence of the invention, such as a sequence given in Table 1 [SEQ ID NO: 1 or 3], typically a library of clones of chromosomal DNA of *Streptococcus pneumoniae* 0100993 in *E.coli* or some other suitable host is probed with a radiolabeled oligonucleotide, preferably a 17-mer or longer, derived from a partial sequence. Clones carrying DNA identical to that of the probe can then be distinguished using stringent conditions. By sequencing the individual clones thus identified with sequencing primers designed from the original sequence it is then possible to extend the sequence in both directions to determine the full gene sequence. Conveniently, such sequencing is performed using denatured double stranded DNA prepared from a plasmid clone. Suitable techniques are described by Maniatis, T., Fritsch, E.F. and Sambrook et al., *MOLECULAR CLONING, A LABORATORY MANUAL,* 2nd Ed.; Cold Spring Harbor Laboratory Press, Cold Spring Harbor, New York (1989). (see in particular Screening By Hybridization 1.90 and Sequencing Denatured Double-Stranded DNA Templates 13.70). Illustrative of the invention, the polynucleotide set out in Table 1 [SEQ ID NO:1 or 3] was discovered in a DNA library derived from *Streptococcus pneumoniae* 0100993.

The DNA sequence set out in Table 1 [SEQ ID NO:1 or 3] contains an open reading frame encoding a protein having about the number of amino acid residues set forth in Table 1 [SEQ ID NO:2 or 4] with a deduced molecular weight that can be calculated using amino acid residue molecular weight values well known in the art. The polynucleotide of SEQ ID NO: 1, between nucleotide number 1 and the stop codon which begins at nucleotide number 2512 of SEQ ID NO: 1, encodes the polypeptide of SEQ ID NO:2.

SecA of the invention is structurally related to other proteins of the SecA family.

The invention provides a polynucleotide sequence identical over its entire length to a coding sequence in Table 1 [SEQ ID NO: 1 or 3]. Also provided by the invention is the coding sequence for the mature polypeptide or a fragment thereof, by itself as well as the coding sequence for the mature polypeptide or a fragment in reading frame with other coding sequence, such as those encoding a leader or secretory sequence, a pre-, or pro- or prepro- protein sequence. The polynucleotide may also contain non-coding sequences, including for example, but not limited to non-coding 5' and 3' sequences, such as the transcribed, non-translated sequences, termination signals, ribosome binding sites, sequences that stabilize mRNA, introns, polyadenylation signals, and additional coding sequence which encode additional amino acids. For example, a marker sequence that facilitates purification of the fused polypeptide can be encoded. In certain embodiments of the invention, the marker sequence is a hexa-histidine peptide, as provided in the pQE vector (Qiagen, Inc.) and described in Gentz *et al., Proc. Natl. Acad. Sci., USA 86:* 821-824 (1989), or an HA tag (Wilson *et al., Cell 37:* 767 (1984). Polynucleotides of the invention also include, but are not limited to, polynucleotides comprising a structural gene and its naturally associated sequences that control gene expression.

A preferred embodiment of the invention is a polynucleotide of comprising nucleotide 1 to the nucleotide immediately upstream of or including nucleotide 2512 set forth in SEQ ID NO: 1 of Table 1, both of which encode the SecA polypeptide.

The invention also includes polynucleotides of the formula:

$$X\text{-}(R_1)_m\text{-}(R_2)\text{-}(R_3)_n\text{-}Y$$

wherein, at the 5' end of the molecule, X is hydrogen or a metal or together with Y defines a covalent bond, and at the 3' end of the molecule, Y is hydrogen or a metal or together with X defines the covalent bond, each occurrence of $R_1$ and $R_3$ is independently any nucleic acid residue, m is an integer between 1 and 3000 or zero , n is an integer between 1 and 3000 or zero, and $R_2$ is a nucleic acid sequence of the invention, particularly a nucleic acid sequence selected from Table 1. In the polynucleotide formula above $R_2$ is oriented so that its 5' end residue is at the left, bound to $R_1$, and its 3' end residue is at the right, bound to $R_3$. Any stretch of nucleic acid residues denoted by either R group, where m and/or n is greater than 1, may be either a heteropolymer or a homopolymer, preferably a heteropolymer. Where, in a preferred embodiment, X and Y together define a covalent bond, the polynucleotide of the above formula is a closed, circular polynucleotide, which can be a double-stranded polynucleotide wherein the formula shows a first strand to which the second strand is complementary. In another preferred embodiment m and/or n is an integer between 1 and 1000.

It is most preferred that the polynucleotides of the inventions are derived from *Streptococcus pneumoniae,* however, they may preferably be obtained from organisms of the same taxonomic genus. They may also be obtained, for example, from organisms of the same taxonomic family or order.

The term "polynucleotide encoding a polypeptide" as used herein encompasses polynucleotides that include a sequence encoding a polypeptide of the invention, particularly a bacterial polypeptide and more particularly a polypeptide of the *Streptococcus pneumoniae* SecA having an amino acid sequence set out in Table 1 [SEQ ID NO:2 or 4]. The term also encompasses polynucleotides that include a single continuous region or discontinuous regions encoding the polypeptide (for example, interrupted by integrated phage or an insertion sequence or editing) together with additional regions, that also may contain coding and/or non-coding sequences.

The invention further relates to variants of the polynucleotides described herein that encode for variants of the polypeptide having a deduced amino acid sequence of Table 1 [SEQ ID NO:2 or 4]. Variants that are fragments of the polynucleotides of the invention may be used to synthesize full-length polynucleotides ofthe invention.

Further particularly preferred embodiments are polynucleotides encoding SecA variants, that have the amino acid sequence of SecA polypeptide of Table 1 [SEQ ID NO:2 or 4] in which several, a few, 5 to 10, 1 to 5, 1 to 3, 2, 1 or no amino acid residues are substituted, deleted or added, in any combination. Especially preferred among these are silent substitutions, additions and deletions, that do not alter the properties and activities of SecA.

Further preferred embodiments of the invention are polynucleotides that are at least 70% identical over their entire length to a polynucleotide encoding SecA polypeptide having an amino acid sequence set out in Table 1 [SEQ ID NO:2 or 4], and polynucleotides that are complementary to such polynucleotides. Alternatively, most highly preferred are polynucleotides that comprise a region that is at least 80% identical over its entire length to a polynucleotide encoding SecA polypeptide and polynucleotides complementary thereto. In this regard, polynucleotides at least 90% identical over their entire length to the same are particularly preferred, and among these particularly preferred polynucleotides, those with at least 95% are especially preferred. Furthermore, those with at least 97% are highly preferred among those with at least 95%, and among these those with at least 98% and at least 99% are particularly highly preferred, with at least 99% being the more preferred.

Preferred embodiments are polynucleotides that encode polypeptides that retain substantially the same biological function or activity as the mature polypeptide encoded by a DNA of Table 1 [SEQ ID NO: 1 or 3].

The invention further relates to polynucleotides that hybridize to the herein above-described sequences. In this regard, the invention especially relates to polynucleotides that hybridize under stringent conditions to the herein above-described polynucleotides. As herein used, the terms "stringent conditions" and "stringent hybridization conditions" mean hybridization will occur only if there is at least 95% and preferably at least 97% identity between the sequences. An example of stringent hybridization conditions is overnight incubation at 42°C in a solution comprising: 50% formamide, 5x SSC (150mM NaCl, 15mM trisodium citrate), 50 mM sodium phosphate (pH7.6), 5x Denhardt's solution, 10% dextran sulfate, and 20 micrograms/ml denatured, sheared salmon sperm DNA, followed by washing the hybridization support in 0.1x SSC at about 65°C. Hybridization and wash conditions are well known and exemplified in Sambrook, *et al.,* Molecular Cloning: A Laboratory Manual, Second Edition, Cold Spring Harbor, N.Y., (1989), particularly Chapter 11 therein.

The invention also provides a polynucleotide consisting essentially of a polynucleotide sequence obtainable by screening an appropriate library containing the complete gene for a polynucleotide sequence set forth in SEQ ID NO:1 under stringent hybridization conditions with a probe having the sequence of said polynucleotide sequence set forth in SEQ ID NO: 1 or a fragment thereof; and isolating said DNA sequence. Fragments useful for obtaining such a polynucleotide include, for example, probes and primers described elsewhere herein.

As discussed additionally herein regarding polynucleotide assays of the invention, for instance, polynucleotides of the invention as discussed above, may be used as a hybridization probe for RNA, cDNA and genomic DNA to isolate full-length cDNAs and genomic clones encoding SecA and to isolate cDNA and genomic clones of other genes that have a high sequence identity to the SecA gene. Such probes generally will comprise at least 15 bases. Preferably, such probes will have at least 30 bases and may have at least 50 bases. Particularly preferred probes will have at least 30 bases and will have 50 bases or less.

For example, the coding region of the SecA gene may be isolated by screening using a DNA sequence provided in Table 1 [SEQ ID NO: 1 or 3] to synthesize an oligonucleotide probe. A labeled oligonucleotide having a sequence complementary to that of a gene of the invention is then used to screen a library of cDNA, genomic DNA or mRNA to determine which members of the library the probe hybridizes to.

The polynucleotides and polypeptides of the invention may be employed, for example, as research reagents and materials for discovery of treatments of and diagnostics for disease, particularly human disease, as further discussed herein relating to polynucleotide assays.

Polynucleotides of the invention that are oligonucleotides derived from the sequences of Table 1 [SEQ ID NOS: 1 or 2 or 3 or 4] may be used in the processes herein as described, but preferably for PCR, to determine whether or not the polynucleotides identified herein in whole or in part are transcribed in bacteria in infected tissue. It is recognized that such sequences will also have utility in diagnosis of the stage of infection and type of infection the pathogen has attained.

The invention also provides polynucleotides that may encode a polypeptide that is the mature protein plus additional amino or carboxyl-terminal amino acids, or amino acids interior to the mature polypeptide (when the mature form has more than one polypeptide chain, for instance). Such sequences may play a role in processing of a protein from precursor to a mature form, may allow protein transport, may lengthen or shorten protein half-life or may facilitate manipulation of a protein for assay or production, among other things. As generally is the case *in vivo,* the additional amino acids may be processed away from the mature protein by cellular enzymes.

A precursor protein, having the mature form of the polypeptide fused to one or more prosequences may be an inactive form of the polypeptide. When prosequences are removed such inactive precursors generally are activated. Some or all of the prosequences may be removed before activation. Generally, such precursors are called proproteins.

In addition to the standard A, G, C, T/U representations for nucleic acid bases, the term "N" may also be used in describing certain polynucleotides of the invention. "N" means that any of the four DNA or RNA bases may appear at such a designated position in the DNA or RNA sequence, except it is preferred that N is not a base that when taken in combination with adjacent nucleotide positions, when read in the correct reading frame, would have the effect of generating a premature termination codon in such reading frame.

In sum, a polynucleotide of the invention may encode a mature protein, a mature protein plus a leader sequence (which may be referred to as a preprotein), a precursor of a mature protein having one or more prosequences that are not the leader sequences of a preprotein, or a preproprotein, which is a precursor to a proprotein, having a leader sequence and one or more prosequences, which generally are removed during processing steps that produce active and mature forms of the polypeptide.

**Vectors, host cells, expression**

The invention also relates to vectors that comprise a polynucleotide or polynucleotides of the invention, host cells that are genetically engineered with vectors of the invention and the production of polypeptides of the invention by recombinant techniques. Cell-free translation systems can also be employed to produce such proteins using RNAs derived from the DNA constructs of the invention.

For recombinant production, host cells can be genetically engineered to incorporate expression systems or portions thereof or polynucleotides of the invention. Introduction of a polynucleotide into the host cell can be effected by methods described in many standard laboratory manuals, such as Davis et al., *BASIC METHODS IN MOLECULAR BIOLOGY,* (1986) and Sambrook et al., *MOLECULAR CLONING: A LABORATORY MANUAL,* 2nd Ed., Cold Spring Harbor Laboratory Press, Cold Spring Harbor, N.Y. (1989), such as, calcium phosphate transfection, DEAE-dextran mediated transfection, transvection, microinjection, cationic lipid-mediated transfection, electroporation, transduction, scrape loading, ballistic introduction and infection.

Representative examples of appropriate hosts include bacterial cells, such as streptococci, staphylococci, enterococci *E. coli,* streptomyces and *Bacillus subtilis* cells; fungal cells, such as yeast cells and *Aspergillus* cells; insect cells such as *Drosophila* S2 and *Spodoptera* Sf9 cells; animal cells such as CHO, COS, HeLa, C127, 3T3, BHK, 293 and Bowes melanoma cells; and plant cells.

A great variety of expression systems can be used to produce the polypeptides of the invention. Such vectors include, among others, chromosomal, episomal and virus-derived vectors, *e.g.,* vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, fowl pox viruses, pseudorabies viruses and retroviruses, and vectors derived from combinations thereof, such as those derived from plasmid and bacteriophage genetic elements, such as cosmids and phagemids. The expression system constructs may contain control regions that regulate as well as engender expression. Generally, any system or vector suitable to maintain, propagate or express polynucleotides and/or to express a polypeptide in a host may be used for expression in this regard. The appropriate DNA sequence may be inserted into the expression system by any of a variety of well-known and routine techniques, such as, for example, those set forth in Sambrook *et al., MOLECULAR CLONING, A LABORATORY MANUAL,* (*supra*).

For secretion of the translated protein into the lumen of the endoplasmic reticulum, into the periplasmic space or into the extracellular environment, appropriate secretion signals may be incorporated into the expressed polypeptide. These signals may be endogenous to the polypeptide or they may be heterologous signals.

Polypeptides of the invention can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography, and lectin chromatography. Most preferably, high performance liquid chromatography is employed for purification. Well known techniques for refolding protein may be employed to regenerate active conformation when the polypeptide is denatured during isolation and or purification.

## Diagnostic, Prognostic, Serotyping and Mutation Assays

This invention is also related to the use of the SecA polynucleotides of the invention for use as diagnostic reagents. Detection of SecA in a eukaryote, particularly a mammal, and especially a human, will provide a diagnostic method for diagnosis of a disease. Eukaryotes (herein also "individual(s)"), particularly mammals, and especially humans, particularly those infected or suspected to be infected with an organism comprising the SecA gene may be detected at the nucleic acid level by a variety of techniques.

Nucleic acids for diagnosis may be obtained from an infected individual's cells and tissues, such as bone, blood, muscle, cartilage, and skin. Genomic DNA may be used directly for detection or may be amplified enzymatically by using PCR or other amplification technique prior to analysis. RNA, cDNA and genomic DNA may also be used in the same ways. Using amplification, characterization of the species and strain of prokaryote present in an individual, may be made by an analysis of the genotype of the prokaryote gene. Deletions and insertions can be detected by a change in size of the amplified product in comparison to the genotype of a reference sequence. Point mutations can be identified by hybridizing amplified DNA to labeled SecA polynucleotide sequences. Perfectly matched sequences can be distinguished from mismatched duplexes by RNase digestion or by differences in melting temperatures. DNA sequence differences may also be detected by alterations in the electrophoretic mobility of the DNA fragments in gels, with or without denaturing agents, or by direct DNA sequencing. See, *e.g.*, Myers et al., *Science, 230:* 1242 (1985). Sequence changes at specific locations also may be revealed by nuclease protection assays, such as RNase and S1 protection or a chemical cleavage method. See, *e.g.,* Cotton et al., *Proc. Natl. Acad Sci., USA, 85:* 4397-4401 (1985).

Cells carrying mutations or polymorphisms (allelic variations) in the gene of the invention may also be detected at the DNA or RNA level by a variety of techniques, to allow for serotyping, for example. For example, RT-PCR can be used to detect mutations in the RNA. It is particularly preferred to used RT-PCR in conjunction with automated detection systems, such as, for example, GeneScan. RNA, cDNA or genomic DNA may also be used for the same purpose, PCR or RT-PCR. As an example, PCR primers complementary to a nucleic acid encoding SecA can be used to identify and analyze mutations. Examples of representative primers are shown below in Table 2.

**Table 2**

**Primers for amplification of SecA polynucleotides**

| SEQ ID NO | PRIMER SEQUENCE |
|---|---|
| 5 | 5'-ATGGCTAATATTTTAAAAAC-3' |
| 6 | 5'-TTATTGTCTTTTACCGTGAC-3' |

The invention also includes primers of the formula:

$$X\text{-}(R_1)_m\text{-}(R_2)\text{-}(R_3)_n\text{-}Y$$

wherein, at the 5' end of the molecule, X is hydrogen or a metal, and at the 3' end of the molecule, Y is hydrogen or a metal, $R_1$ and $R_3$ is any nucleic acid residue, m is an integer between 1 and 20 or zero , n is an integer between 1 and 20 or zero, and $R_2$ is a primer sequence of the invention, particularly a primer sequence selected from Table 2. In the polynucleotide formula above $R_2$ is oriented so that its 5' end residue is at the left, bound to $R_1$, and its 3' end residue is at the right, bound to $R_3$. Any stretch of nucleic acid residues denoted by either R group, where m and/or n is greater than 1, may be either a heteropolymer or a homopolymer, preferably a heteropolymer being complementary to a region of a polynucleotide of Table 1. In a preferred embodiment m and/or n is an integer between 1 and 10.

The invention further provides these primers with 1, 2, 3 or 4 nucleotides removed from the 5' and/or the 3' end. These primers may be used for, among other things, amplifying SecA DNA isolated from a sample derived from an individual. The primers may be used to amplify the gene isolated from an infected individual such that the gene may then be subject to various techniques for elucidation of the DNA sequence. In this way, mutations in the DNA sequence may be detected and used to diagnose infection and to serotype and/or classify the infectious agent.

The invention further provides a process for diagnosing, disease, preferably bacterial infections, more preferably infections by *Streptococcus pneumoniae,* comprising determining from a sample derived from an individual a increased level of expression of polynucleotide having a sequence of Table 1 [SEQ ID NO: 1 or 3]. Increased or decreased expression of SecA polynucleotide can be measured using any on of the methods well known in the art for the quantitation of polynucleotides, such as, for example, amplification, PCR, RT-PCR, RNase protection, Northern blotting and other hybridization methods.

In addition, a diagnostic assay in accordance with the invention for detecting over-expression of SecA protein compared to normal control tissue samples may be used to detect the presence of an infection, for example. Assay techniques that can be used to determine levels of a SecA protein, in a sample derived from a host are well-known to those of skill in the art. Such assay methods include radioimmunoassays, competitive-binding assays, Western Blot analysis and ELISA assays.

The polynucleotide sequences of the present invention are also valuable for chromosome identification. The sequence is specifically targeted to, and can hybridize with, a particular location on an individual microbial chromosome, particularly a Streptococcus pneumoniae chromosome. The mapping of relevant sequences to a chromosome according to the present invention is an important first step in correlating those sequences with gene associated with microbial pathogenicity and disease, or to chromosomal regions critical to the growth, survival and/or ecological niche. Once a sequence has been mapped to a precise chromosomal location, the physical position of the sequence on the chromosome can be correlated with genetic map data. Such data are found in, for example, microbial genomic sequences available on the World Wide Web. The relationship between genes and microbial pathogenicity, disease, or to genome regions critical to the growth, survival and/or ecological niche that have been mapped to the same chromosomal region are then identified using methods to define a genetic relationship between the gene and another gene or phenotype, such as by linkage analysis (coinheritance of physically adjacent genes).

The differences in the RNA or genomic sequence between microbes of differing phenotypes can also be determined. If a mutation or sequence is observed in some or all of the microbes of a certain phenotype, but not in any microbes lacking that phenotype, then the mutation or sequence is likely to be the causative agent of the phenotype. In this way, chromosomal regions may be identified that confer microbial pathogenicity, growth characteristics, survival characteristics and/or ecological niche characteristics.

**Differential Expression**

The polynucleotides and polynucleotides of the invention may be used as reagents for differential screening methods. There are many differential screening and differential display methods known in the art in which the polynucleotides and polypeptides of the invention may be used. For example, the differential display technique is described by Chuang *et al., J. Bacteriol. 175:*2026-2036 (1993). This method identifies those genes which are expressed in an organism by identifying mRNA present using randomly-primed RT-PCR. By comparing pre-infection and post infection profiles, genes up and down regulated during infection can be identified and the RT-PCR product sequenced and matched to ORF 'unknowns'.

In Vivo Expression Technology (IVET) is described by Camilli *et al., Proc. Nat'l. Acad. Sci. USA. 91:*2634-2638 (1994). IVET identifies genes up-regulated during infection when compared to laboratory cultivation, implying an important role in infection. ORF identified by this technique are implied to have a significant role in infection establishment and/or maintenance. In this technique random chromosomal fragments of target organism are cloned upstream of a promoter-less recombinase gene in a plasmid vector. This construct is introduced into the target organism which carries an antibiotic resistance gene flanked by resolvase sites. Growth in the presence of the antibiotic removes from the population those fragments cloned into the plasmid vector capable of supporting transcription of the recombinase gene and therefore have caused loss of antibiotic resistance. The resistant pool is introduced into a host and at various times after infection bacteria may be recovered and assessed for the presence of antibiotic resistance. The chromosomal fragment carried by each antibiotic sensitive bacterium should carry a promoter or portion of a gene normally upregulated during infection. Sequencing upstream of the recombinase gene allows identification of the up regulated gene.

RT-PCR may also be used to analyze gene expression patterns. For RT PCR using the polynucleotides of the invention, messenger RNA is isolated from bacterial infected tissue, e.g., 48 hour murine lung infections, and the amount of each mRNA species assessed by reverse transcription of the RNA sample primed with random hexanucleotides followed by PCR with gene specific primer pairs. The determination of the presence and amount of a particular mRNA species by quantification of the resultant PCR product provides information on the bacterial genes which are transcribed in the infected tissue. Analysis of gene transcription can be carried out at different times of infection to gain a detailed knowledge of gene regulation in bacterial pathogenesis allowing for a clearer understanding of which gene products represent targets for screens for antibacterials. Because of the gene specific nature of the PCR primers employed it should be understood that the bacterial mRNA preparation need not be free of mammalian RNA. This allows the investigator to carry out a simple and quick RNA preparation from infected tissue to obtain bacterial mRNA species which are very short lived in the bacterium (in the order of 2 minute halflives). Optimally the bacterial mRNA is prepared from infected murine lung tissue by mechanical disruption in the presence of TRIzole (GIBCO-BRL) for very short periods of time, subsequent processing according to the manufacturers of TRIzole reagent and DNAase treatment to remove contaminating DNA. Preferably the process is optimised by finding those conditions which give a maximum amount of Streptococcus pneumoniae 16S ribosomal RNA as detected by probing Northerns with a suitably labelled sequence specific oligonucleotide probe. Typically a 5' dye labelled primer is used in each PCR primer pair in a PCR reaction which is terminated optimally between 8 and 25 cycles. The PCR products are separated on 6% polyacrylamide gels with detection and quantification using Gene Scanner (manufactured by ABI).

Each of these techniques may have advantages or disadvantage depending on the particular application. The skilled artisan would choose the approach that is the most relevant with the particular end use in mind.

## Antibodies

The polypeptides of the invention or variants thereof, or cells expressing them can be used as an immunogen to produce antibodies immunospecific for such polypeptides. "Antibodies" as used herein includes monoclonal and polyclonal antibodies, chimeric, single chain, simianized antibodies and humanized antibodies, as well as Fab fragments, including the products of an Fab immunolglobulin expression library.

Antibodies generated against the polypeptides of the invention can be obtained by administering the polypeptides or epitope-bearing fragments, analogues or cells to an animal, preferably a nonhuman, using routine protocols. For preparation of monoclonal antibodies, any technique known in the art that provides antibodies produced by continuous cell line cultures can be used. Examples include various techniques, such as those in Kohler, G. and Milstein, C., *Nature 256:* 495-497 (1975); Kozbor *et al., Immunology Today 4:* 72 (1983); Cole et al., pg. 77-96 in *MONOCLONAL ANTIBODIES AND CANCER THERAPY,* Alan R. Liss, Inc. (1985).

Techniques for the production of single chain antibodies (U.S. Patent No. 4,946,778) can be adapted to produce single chain antibodies to polypeptides of this invention. Also, transgenic mice, or other organisms such as other mammals, may be used to express humanized antibodies.

Alternatively phage display technology may be utilized to select antibody genes with binding activities towards the polypeptide either from repertoires of PCR amplified v-genes of lymphocytes from humans screened for possessing anti-SecA or from naive libraries (McCafferty, J. et al., (1990), Nature 348, 552-554; Marks, J. et al., (1992) Biotechnology *10,* 779-783). The affinity of these antibodies can also be improved by chain shuffling (Clackson, T. et al., (1991) Nature 352, 624-628).

If two antigen binding domains are present each domain may be directed against a different epitope - termed 'bispecific' antibodies.

The above-described antibodies may be employed to isolate or to identify clones expressing the polypeptides to purify the polypeptides by affinity chromatography.

Thus, among others, antibodies against SecA-polypeptide may be employed to treat infections, particularly bacterial infections.

Polypeptide variants include antigenically, epitopically or immunologically equivalent variants that form a particular aspect of this invention. The term "antigenically equivalent derivative" as used herein encompasses a polypeptide or its equivalent which will be specifically recognized by certain antibodies which, when raised to the protein or polypeptide according to the invention, interfere with the immediate physical interaction between pathogen and mammalian host. The term "immunologically equivalent derivative" as used herein encompasses a peptide or its equivalent which when used in a suitable formulation to raise antibodies in a vertebrate, the antibodies act to interfere with the immediate physical interaction between pathogen and mammalian host.

The polypeptide, such as an antigenically or immunologically equivalent derivative or a fusion protein thereof is used as an antigen to immunize a mouse or other animal such as a rat or chicken. The fusion protein may provide stability to the polypeptide. The antigen may be associated, for example by conjugation, with an immunogenic carrier protein for example bovine serum albumin (BSA) or keyhole limpet haemocyanin (KLH). Alternatively a multiple antigenic peptide comprising multiple copies of the protein or polypeptide, or an antigenically or immunologically equivalent polypeptide thereof may be sufficiently antigenic to improve immunogenicity so as to obviate the use of a carrier.

Preferably, the antibody or variant thereof is modified to make it less immunogenic in the individual. For example, if the individual is human the antibody may most preferably be "humanized"; where the complimentarity determining region(s) of the hybridoma-derived antibody has been transplanted into a human monoclonal antibody, for example as described in Jones, P. *et al.* (1986), *Nature* 321, 522-525 or Tempest *et al.,* (1991) *Biotechnology* 9, 266-273.

The use of a polynucleotide of the invention in genetic immunization will preferably employ a suitable delivery method such as direct injection of plasmid DNA into muscles (Wolff et al., Hum Mol Genet 1992, 1:363, Manthorpe et al., Hum. Gene Ther. 1963:4, 419), delivery of DNA complexed with specific protein carriers (Wu et al., *J Biol Chem.* 1989: 264,16985), coprecipitation of DNA with calcium phosphate (Benvenisty & Reshef, *PNAS USA,* 1986:83,9551), encapsulation of DNA in various forms of liposomes (Kaneda et al., *Science* 1989:243,375), particle bombardment (Tang et al., *Nature* 1992, 356:152, Eisenbraun et al., *DNA Cell Biol* 1993, 12:791) and *in vivo* infection using cloned retroviral vectors (Seeger et al., *PNAS USA* 1984:81,5849).

**Antagonists and agonists - assays and molecules**

Polypeptides of the invention may also be used to assess the binding of small molecule substrates and ligands in, for example, cells, cell-free preparations, chemical libraries, and natural product mixtures. These substrates and ligands may be natural substrates and ligands or may be structural or functional mimetics. See, *e.g.,* Coligan *et al., Current Protocols in Immunology 1(2):* Chapter 5 (1991).

The invention also provides a method of screening compounds to identify those which enhance (agonist) or block (antagonist) the action of SecA polypeptides or polynucleotides, particularly those compounds that are bacteriostatic and/or bacteriocidal. The method of screening may involve high-throughput techniques. For example, to screen for agonists or antagonists, a synthetic reaction mix, a cellular compartment, such as a membrane, cell envelope or cell wall, or a preparation of any thereof, comprising SecA polypeptide and a labeled substrate or ligand of such polypeptide is incubated in the absence or the presence of a candidate molecule that may be a SecA agonist or antagonist. The ability of the candidate molecule to agonize or antagonize the SecA polypeptide is reflected in decreased binding of the labeled ligand or decreased production of product from such substrate. Molecules that bind gratuitously, *i.e.,* without inducing the effects of SecA polypeptide are most likely to be good antagonists. Molecules that bind well and increase the rate of product production from substrate are agonists. Detection of the rate or level of production of product from substrate may be enhanced by using a reporter system. Reporter systems that may be useful in this regard include but are not limited to colorimetric labeled substrate converted into product, a reporter gene that is responsive to changes in SecA polynucleotide or polypeptide activity, and binding assays known in the art.

Another example of an assay for SecA antagonists is a competitive assay that combines SecA and a potential antagonist with SecA-binding molecules, recombinant SecA binding molecules, natural substrates or ligands, or substrate or ligand mimetics, under appropriate conditions for a competitive inhibition assay. SecA can be labeled, such as by radioactivity or a colorimetric compound, such that the number of SecA molecules bound to a binding molecule or converted to product can be determined accurately to assess the effectiveness of the potential antagonist.

Potential antagonists include small organic molecules, peptides, polypeptides and antibodies that bind to a polynucleotide or polypeptide of the invention and thereby inhibit or extinguish its activity. Potential antagonists also may be small organic molecules, a peptide, a polypeptide such as a closely related protein or antibody that binds the same sites on a binding molecule, such as a binding molecule, without inducing SecA-induced activities, thereby preventing the action of SecA by excluding SecA from binding.

Potential antagonists include a small molecule that binds to and occupies the binding site of the polypeptide thereby preventing binding to cellular binding molecules, such that normal biological activity is prevented. Examples of small molecules include but are not limited to small organic molecules, peptides or peptide-like molecules. Other potential antagonists include antisense molecules (see Okano, *J. Neurochem. 56:* 560 (1991); *OLIGODEOXYNUCLEOTIDES AS ANTISENSE INHIBITORS OF GENE EXPRESSION,* CRC Press, Boca Raton, FL (1988), for a description of these molecules). Preferred potential antagonists include compounds related to and variants of SecA.

Each of the DNA sequences provided herein may be used in the discovery and development of antibacterial compounds. The encoded protein, upon expression, can be used as a target for the screening of antibacterial drugs. Additionally, the DNA sequences encoding the amino terminal regions of the encoded protein or Shine-Delgarno or other translation facilitating sequences of the respective mRNA can be used to construct antisense sequences to control the expression of the coding sequence of interest.

The invention also provides the use of the polypeptide, polynucleotide or inhibitor of the invention to interfere with the initial physical interaction between a pathogen and mammalian host responsible for sequelae of infection. In particular the molecules of the invention may be used: in the prevention of adhesion of bacteria, in particular gram positive bacteria, to mammalian extracellular matrix proteins on in-dwelling devices or to extracellular matrix proteins in wounds; to block SecA protein-mediated mammalian cell invasion by, for example, initiating phosphorylation of mammalian tyrosine kinases (Rosenshine *et al., Infect. Immun. 60:*2211 (1992); to block bacterial adhesion between mammalian extracellular matrix proteins and bacterial SecA proteins that mediate tissue damage and; to block the normal progression of pathogenesis in infections initiated other than by the implantation of in-dwelling devices or by other surgical techniques.

The antagonists and agonists of the invention may be employed, for instance, to inhibit and treat diseases.

*Helicobacter pylori* (herein *H. pylori)* bacteria infect the stomachs of over one-third of the world's population causing stomach cancer, ulcers, and gastritis (International Agency for Research on Cancer (1994) Schistosomes, Liver Flukes and Helicobacter Pylori (International Agency for Research on Cancer, Lyon, France; http://www.uicc.ch/ecp/ecp2904.htm). Moreover, the international Agency for Research on Cancer recently recognized a cause-and-effect relationship between *H. pylori* and gastric adenocarcinoma, classifying the bacterium as a Group I (definite) carcinogen. Preferred antimicrobial compounds of the invention (agonists and antagonists of SecA) found using screens provided by the invention, particularly broad-spectrum antibiotics, should be useful in the treatment of *H. pylori* infection. Such treatment should decrease the advent of *H. pylori*-induced cancers, such as gastrointestinal carcinoma. Such treatment should also cure gastric ulcers and gastritis.

**Vaccines**

Another aspect of the invention relates to a method for inducing an immunological response in an individual, particularly a mammal which comprises inoculating the individual with SecA, or a fragment or variant thereof, adequate to produce antibody and/ or T cell immune response to protect said individual from infection, particularly bacterial infection and most particularly *Streptococcus pneumoniae* infection. Also provided are methods whereby such immunological response slows bacterial replication. Yet another aspect of the invention relates to a method of inducing immunological response in an individual which comprises delivering to such individual a nucleic acid vector to direct expression of SecA, or a fragment or a variant thereof, for expressing SecA, or a fragment or a variant thereof *in vivo* in order to induce an immunological response, such as, to produce antibody and/ or T cell immune response, including, for example, cytokine-producing T cells or cytotoxic T cells, to protect said individual from disease, whether that disease is already established within the individual or not. One way of administering the gene is by accelerating it into the desired cells as a coating on particles or otherwise. Such nucleic acid vector may comprise DNA, RNA, a modified nucleic acid, or a DNA/RNA hybrid.

A further aspect of the invention relates to an immunological composition which, when introduced into an individual capable or having induced within it an immunological response, induces an immunological response in such individual to a SecA or protein coded therefrom, wherein the composition comprises a recombinant SecA or protein coded therefrom comprising DNA which codes for and expresses an antigen of said SecA or protein coded therefrom. The immunological response may be used therapeutically or prophylactically and may take the form of antibody immunity or cellular immunity such as that arising from CTL or CD4+ T cells.

A SecA polypeptide or a fragment thereof may be fused with co-protein which may not by itself produce antibodies, but is capable of stabilizing the first protein and producing a fused protein which will have immunogenic and protective properties. Thus fused recombinant protein, preferably further comprises an antigenic co-protein, such as lipoprotein D from *Hemophilus influenzae,* Glutathione-S-transferase (GST) or beta-galactosidase, relatively large co-proteins which solubilize the protein and facilitate production and purification thereof. Moreover, the co-protein may act as an adjuvant in the sense of providing a generalized stimulation of the immune system. The co-protein may be attached to either the amino or carboxy terminus of the first protein.

Provided by this invention are compositions, particularly vaccine compositions, and methods comprising the polypeptides or polynucleotides of the invention and immunostimulatory DNA sequences, such as those described in Sato, Y. *et al.* Science 273: 352 (1996).

Also, provided by this invention are methods using the described polynucleotide or particular fragments thereof which have been shown to encode non-variable regions of bacterial cell surface proteins in DNA constructs used in such genetic immunization experiments in animal models of infection with *Streptococcus pneumoniae* will be particularly useful for identifying protein epitopes able to provoke a prophylactic or therapeutic immune response. It is believed that this approach will allow for the subsequent preparation of monoclonal antibodies of particular value from the requisite organ of the animal successfully resisting or clearing infection for the development of prophylactic agents or therapeutic treatments of bacterial infection, particularly *Streptococcus pneumoniae* infection, in mammals, particularly humans.

The polypeptide may be used as an antigen for vaccination of a host to produce specific antibodies which protect against invasion of bacteria, for example by blocking adherence of bacteria to damaged tissue. Examples of tissue damage include wounds in skin or connective tissue caused, e.g., by mechanical, chemical or thermal damage or by implantation of indwelling devices, or wounds in the mucous membranes, such as the mouth, mammary glands, urethra or vagina.

The invention also includes a vaccine formulation which comprises an immunogenic recombinant protein of the invention together with a suitable carrier. Since the protein may be broken down in the stomach, it is preferably administered parenterally, including, for example, administration that is subcutaneous, intramuscular, intravenous, or intradermal. Formulations suitable for parenteral administration include aqueous and non-aqueous sterile injection solutions which may contain anti-oxidants, buffers, bacteriostats and solutes which render the formulation insotonic with the bodily fluid, preferably the blood, of the individual; and aqueous and non-aqueous sterile suspensions which may include suspending agents or thickening agents. The formulations may be presented in unit-dose or multi-dose containers, for example, sealed ampules and vials and may be stored in a freeze-dried condition requiring only the addition of the sterile liquid carrier immediately prior to use. The vaccine formulation may also include adjuvant systems for enhancing the immunogenicity of the formulation, such as oil-in water systems and other systems known in the art. The dosage will depend on the specific activity of the vaccine and can be readily determined by routine experimentation.

While the invention has been described with reference to certain SecA protein, it is to be understood that this covers fragments of the naturally occurring protein and similar proteins with additions, deletions or substitutions which do not substantially affect the immunogenic properties of the recombinant protein.

**Compositions, kits and administration**

The invention also relates to compositions comprising the polynucleotide or the polypeptides discussed above or their agonists or antagonists. The polypeptides of the invention may be employed in combination with a non-sterile or sterile carrier or carriers for use with cells, tissues or organisms, such as a pharmaceutical carrier suitable for administration to a subject. Such compositions comprise, for instance, a media additive or a therapeutically effective amount of a polypeptide of the invention and a pharmaceutically acceptable carrier or excipient. Such carriers may include, but are not limited to, saline, buffered saline, dextrose, water, glycerol, ethanol and combinations thereof. The formulation should suit the mode of administration. The invention further relates to diagnostic and pharmaceutical packs and kits comprising one or more containers filled with one or more of the ingredients of the aforementioned compositions of the invention.

Polypeptides and other compounds of the invention may be employed alone or in conjunction with other compounds, such as therapeutic compounds.

The pharmaceutical compositions may be administered in any effective, convenient manner including, for instance, administration by topical, oral, anal, vaginal, intravenous, intraperitoneal, intramuscular, subcutaneous, intranasal or intradermal routes among others.

In therapy or as a prophylactic, the active agent may be administered to an individual as an injectable composition, for example as a sterile aqueous dispersion, preferably isotonic.

Alternatively the composition may be formulated for topical application for example in the form of ointments, creams, lotions, eye ointments, eye drops, ear drops, mouthwash, impregnated dressings and sutures and aerosols, and may contain appropriate conventional additives, including, for example, preservatives, solvents to assist drug penetration, and emollients in ointments and creams. Such topical formulations may also contain compatible conventional carriers, for example cream or ointment bases, and ethanol or oleyl alcohol for lotions. Such carriers may constitute from about 1% to about 98% by weight of the formulation; more usually they will constitute up to about 80% by weight of the formulation.

For administration to mammals, and particularly humans, it is expected that the daily dosage level of the active agent will be from 0.01 mg/kg to 10 mg/kg, typically around 1 mg/kg. The physician in any event will determine the actual dosage which will be most suitable for an individual and will vary with the age, weight and response of the particular individual. The above dosages are exemplary of the average case. There can, of course, be individual instances where higher or lower dosage ranges are merited, and such are within the scope of this invention.

In-dwelling devices include surgical implants, prosthetic devices and catheters, i.e., devices that are introduced to the body of an individual and remain in position for an extended time. Such devices include, for example, artificial joints, heart valves, pacemakers, vascular grafts, vascular catheters, cerebrospinal fluid shunts, urinary catheters, continuous ambulatory peritoneal dialysis (CAPD) catheters.

The composition of the invention may be administered by injection to achieve a systemic effect against relevant bacteria shortly before insertion of an in-dwelling device. Treatment may be continued after surgery during the in-body time of the device. In addition, the composition could also be used to broaden perioperative cover for any surgical technique to prevent bacterial wound infections, especially *Streptococcus pneumoniae* wound infections.

Many orthopaedic surgeons consider that humans with prosthetic joints should be considered for antibiotic prophylaxis before dental treatment that could produce a bacteremia. Late deep infection is a serious complication sometimes leading to loss of the prosthetic joint and is accompanied by significant morbidity and mortality. It may therefore be possible to extend the use of the active agent as a replacement for prophylactic antibiotics in this situation.

In addition to the therapy described above, the compositions of this invention may be used generally as a wound treatment agent to prevent adhesion of bacteria to matrix proteins exposed in wound tissue and for prophylactic use in dental treatment as an alternative to, or in conjunction with, antibiotic prophylaxis.

Alternatively, the composition of the invention may be used to bathe an indwelling device immediately before insertion. The active agent will preferably be present at a concentration of 1µg/ml to 10mg/ml for bathing of wounds or indwelling devices.

A vaccine composition is conveniently in injectable form. Conventional adjuvants may be employed to enhance the immune response. A suitable unit dose for vaccination is 0.5-5 microgram/kg of antigen, and such dose is preferably administered 1-3 times and with an interval of 1-3 weeks. With the indicated dose range, no adverse toxicological effects will be observed with the compounds of the invention which would preclude their administration to suitable individuals.

**Sequence Databases and Algorithms**

The polynucleotide and polypeptide sequences of the invention are particularly useful as components in databases useful for search analyses as well as in sequence analysis algorithms. As used in this section entitled Databases and Algorithms and in claims related thereto, the terms "polynucleotide of the invention" and "polynucleotide sequence of the invention" mean any detectable chemical or physical characteristic of a polynucleotide of the invention that is or may be reduced to or stored in a computer readable form. For example, chromatographic scan data or peak data, photographic data or scan data therefrom, called bases, and mass spectrographic data. As used in this section entitled Databases and Algorithms and in claims related thereto, the terms "polypeptide of the invention" and "polypeptide sequence of the invention" mean any detectable chemical or physical characteristic of a polypeptide of the invention that is or may be reduced to or stored in a computer readable form. For example, chromatographic scan data or peak data, photographic data or scan data therefrom, and mass spectrographic data.

The invention provides computer readable medium having stored thereon sequences of the invention. For example, a computer readable medium is provided having stored thereon a member selected from the group consisting of: a polynucleotide comprising the sequence of a polynucleotide of the invention; a polypeptide comprising the sequence of a polypeptide sequence of the invention; a set of polynucleotide sequences wherein at least one of said sequences comprises the sequence of a polynucleotide sequence of the invention; a set of polypeptide sequences wherein at least one of said sequences comprises the sequence of a polypeptide sequence of the invention; a data set representing a polynucleotide sequence comprising the sequence of polynucleotide sequence of the invention; a data set representing a polynucleotide sequence encoding a polypeptide sequence comprising the sequence of a polypeptide sequence of the invention; a polynucleotide comprising the sequence of a polynucleotide sequence of the invention; a polypeptide comprising the sequence of a polypeptide sequence of the invention; a set of polynucleotide sequences wherein at least one of said sequences comprises the sequence of a polynucleotide sequence of the invention; a set of polypeptide sequences wherein at least one of said sequences comprises the sequence of a polypeptide sequence of the invention; a data set representing a polynucleotide sequence comprising the sequence of a polynucleotide sequence of the invention; a data set representing a polynucleotide sequence encoding a polypeptide sequence comprising the sequence of a polypeptide sequence of the invention. The computer readable medium can be any composition of matter used to store information or data, including, for example, commercially available floppy disks, tapes, hard drives, compact disks, and video disks.

Also provided by the invention are methods for the analysis of character sequences, particularly genetic sequences. Preferred methods of sequence analysis include, for example, methods of sequence homology analysis, such as identity and similarity analysis, RNA structure analysis, sequence assembly, cladistic analysis, sequence motif analysis, open reading frame determination, nucleic acid base calling, and sequencing chromatogram peak analysis.

A computer based method is provided for performing homology identification. This method comprises the steps of providing a polynucleotide sequence comprising the sequence a polynucleotide of the invention in a computer readable medium; and comparing said polynucleotide sequence to at least one polynucleotide or polypeptide sequence to identify homology.

A computer based method is also provided for performing homology identification, said method comprising the steps of: providing a polypeptide sequence comprising the sequence of a polypeptide of the invention in a computer readable medium; and comparing said polypeptide sequence to at least one polynucleotide or polypeptide sequence to identify homology.

A computer based method is still further provided for polynucleotide assembly, said method comprising the steps of: providing a first polynucleotide sequence comprising the sequence of a polynucleotide of the invention in a computer readable medium; and screening for at least one overlapping region between said first polynucleotide sequence and a second polynucleotide sequence.

A further embodiment of the invention provides a computer based method for performing homology identification, said method comprising the steps of: providing a polynucleotide sequence comprising the sequence of a polynucleotide of the invention in a computer readable medium; and comparing said polynucleotide sequence to at least one polynucleotide or polypeptide sequence to identify homology.

A further embodiment of the invention provides a computer based method for performing homology identification, said method comprising the steps of: providing a polypeptide sequence comprising the sequence of a polypeptide of the invention in a computer readable medium; and comparing said polypeptide sequence to at least one polynucleotide or polypeptide sequence to identify homology.

A further embodiment of the invention provides a computer based method for polynucleotide assembly, said method comprising the steps of: providing a first polynucleotide sequence comprising the sequence of a polynucleotide of the invention in a computer readable medium; and screening for at least one overlapping region between said first polynucleotide sequence and a second polynucleotide sequence.
Each reference disclosed herein is incorporated by reference herein in its entirety. Any patent application to which this application claims priority is also incorporated by reference herein in its entirety.

## GLOSSARY

The following definitions are provided to facilitate understanding of certain terms used frequently herein.
"Disease(s)" means and disease caused by or related to infection by a bacteria, including otitis media, conjunctivitis, pneumonia, bacteremia, meningitis, sinusitis, pleural empyema and endocarditis, and most particularly meningitis, such as for example infection of cerebrospinal fluid.
"Host cell" is a cell which has been transformed or transfected, or is capable of transformation or transfection by an exogenous polynucleotide sequence.
"Identity," as known in the art, is a relationship between two or more polypeptide sequences or two or more polynucleotide sequences, as the case may be, as determined by comparing the sequences. In the art, "identity" also means the degree of sequence relatedness between polypeptide or polynucleotide sequences, as the case may be, as determined by the match between strings of such sequences. "Identity" can be readily calculated by known methods, including but not limited to those described in (*Computational Molecular Biology,* Lesk, A.M., ed., Oxford University Press, New York, 1988; *Biocomputing: Informatics and Genome Projects,* Smith, D.W., ed., Academic Press, New York, 1993; *Computer Analysis of Sequence Data,* Part I, Griffin, A.M., and Griffin, H.G., eds., Humana Press, New Jersey, 1994; *Sequence Analysis in Molecular Biology,* von Heinje, G., Academic Press, 1987; and *Sequence Analysis Primer,* Gribskov, M. and Devereux, J., eds., M Stockton Press, New York, 1991; and Carillo, H., and Lipman, D., SIAM *J. Applied Math., 48:* 1073 (1988). Methods to determine identity are designed to give the largest match between the sequences tested. Moreover, methods to determine identity are codified in publicly available computer programs. Computer program methods to determine identity between two sequences include, but are not limited to, the GCG program package (Devereux, J., et al., *Nucleic Acids Research 12(1):* 387 (1984)), BLASTP, BLASTN, and FASTA (Atschul, S. F. et al., *J. Molec. Biol. 215:* 403-410 (1990). The BLAST X program is publicly available from NCBI and other sources *(BLAST Manual,* Altschul, S., *et al.,* NCBI NLM NIH Bethesda, MD 20894; Altschul, S., *et al., J. Mol. Biol. 215*: 403-410 (1990). The well known Smith Waterman algorithm may also be used to determine identity.
Parameters for polypeptide sequence comparison include the following:

1) Algorithm: Needleman and Wunsch, J. Mol Biol. 48: 443-453 (1970)
Comparison matrix: BLOSSUM62 from Hentikoff and Hentikoff, Proc. Natl. Acad. Sci. USA. 89:10915-10919 (1992)
Gap Penalty: 12
Gap Length Penalty: 4

A program useful with these parameters is publicly available as the "gap" program from Genetics Computer Group, Madison WI. The aforementioned parameters are the default parameters for peptide comparisons (along with no penalty for end gaps).
Parameters for polynucleotide comparison include the following:

1) Algorithm: Needleman and Wunsch, J. Mol Biol. 48: 443-453 (1970)
Comparison matrix: matches = +10, mismatch = 0
Gap Penalty: 50
Gap Length Penalty: 3

Available as: The "gap" program from Genetics Computer Group, Madison WI. These are the default parameters for nucleic acid comparisons.
A preferred meaning for "identity" for polynucleotides and polypeptides, as the case may be, are provided in (1) and (2) below.

(1) Polynucleotide embodiments further include an isolated polynucleotide comprising a polynucleotide sequence having at least a 50, 60, 70, 80, 85, 90, 95, 97 or 100% identity to the reference sequence of SEQ ID NO: 1, wherein said polynucleotide sequence may be identical to the reference sequence of SEQ ID NO: 1 or may include up to a certain integer number of nucleotide alterations as compared to the reference sequence, wherein said alterations are selected from the group consisting of at least one nucleotide deletion, substitution, including transition and transversion, or insertion, and wherein said alterations may occur at the 5' or 3' terminal positions of the reference nucleotide sequence or anywhere between those terminal positions, interspersed either individually among the nucleotides in the reference sequence or in one or more contiguous groups within the reference sequence, and wherein said number of nucleotide alterations is determined by multiplying the total number of nucleotides in SEQ ID NO: 1 by the integer defining the percent identity divided by 100 and then subtracting that product from said total number of nucleotides in SEQ ID NO: 1, or:

$$n_n \leq x_n - (x_n \bullet y),$$

wherein $\mathbf{n_n}$ is the number of nucleotide alterations, $\mathbf{x_n}$ is the total number of nucleotides in SEQ ID NO: 1, **y** is 0.50 for 50%, 0.60 for 60%, 0.70 for 70%, 0.80 for 80%, 0.85 for 85%, 0.90 for 90%, 0.95 for 95%, 0.97 for 97% or 1.00 for 100%, and • is the symbol for the multiplication operator, and wherein any non-integer product of $\mathbf{x_n}$ and y is rounded down to the nearest integer prior to subtracting it from $\mathbf{x_n}$. Alterations of a polynucleotide sequence encoding the polypeptide of SEQ ID NO:2 may create nonsense, missense or frameshift mutations in this coding sequence and thereby alter the polypeptide encoded by the polynucleotide following such alterations.

By way of example, a polynucleotide sequence of the present invention may be identical to the reference sequence of SEQ ID NO:2, that is it may be 100% identical, or it may include up to a certain integer number of amino acid alterations as compared to the reference sequence such that the percent identity is less than 100% identity. Such alterations are selected from the group consisting of at least one nucleic acid deletion, substitution, including transition and transversion, or insertion, and wherein said alterations may occur at the 5' or 3' terminal positions of the reference polynucleotide sequence or anywhere between those terminal positions, interspersed either individually among the nucleic acids in the reference sequence or in one or more contiguous groups within the reference sequence. The number of nucleic acid alterations for a given percent identity is determined by multiplying the total number of amino acids in SEQ ID NO:2 by the integer defining the percent identity divided by 100 and then subtracting that product from said total number of amino acids in SEQ ID NO:2, or:

$$n_n \leq x_n - (x_n \bullet y),$$

wherein $n_n$ is the number of amino acid alterations, $\mathbf{x_n}$ is the total number of amino acids in SEQ ID NO:2, **y** is, for instance 0.70 for 70%, 0.80 for 80%, 0.85 for 85% etc., • is the symbol for the multiplication operator, and wherein any non-integer product of $\mathbf{x_n}$ and **y** is rounded down to the nearest integer prior to subtracting it from $\mathbf{x_n}$.

(2) Polypeptide embodiments further include an isolated polypeptide comprising a polypeptide having at least a 50,60, 70, 80, 85, 90, 95, 97 or 100% identity to a polypeptide reference sequence of SEQ ID NO:2, wherein said polypeptide sequence may be identical to the reference sequence of SEQ ID NO: 2 or may include up to a certain integer number of amino acid alterations as compared to the reference sequence, wherein said alterations are selected from the group consisting of at least one amino acid deletion, substitution, including conservative and non-conservative substitution, or insertion, and wherein said alterations may occur at the amino- or carboxy-terminal positions of the reference polypeptide sequence or anywhere between those terminal positions, interspersed either individually among the amino acids in the reference sequence or in one or more contiguous groups within the reference sequence, and wherein said number of amino acid alterations is determined by multiplying the total number of amino acids in SEQ ID NO:2 by the integer defining the percent identity divided by 100 and then subtracting that product from said total number of amino acids in SEQ ID NO:2, or:

$$n_a \leq x_a - (x_a \bullet y),$$

wherein $\mathbf{n_a}$ is the number of amino acid alterations, $\mathbf{x_a}$ is the total number of amino acids in SEQ ID NO:2, **y** is 0.50 for 50%, 0.60 for 60%, 0.70 for 70%, 0.80 for 80%, 0.85 for 85%, 0.90 for 90%, 0.95 for 95%, 0.97 for 97% or 1.00 for 100%, and • is the symbol for the multiplication operator, and wherein any non-integer product of $\mathbf{x_a}$ and **y** is rounded down to the nearest integer prior to subtracting it from $\mathbf{x_a}$.

By way of example, a polypeptide sequence of the present invention may be identical to the reference sequence of SEQ ID NO:2, that is it may be 100% identical, or it may include up to a certain integer number of amino acid alterations as compared to the reference sequence such that the percent identity is less than 100% identity. Such alterations are selected from the group consisting of at least one amino acid deletion, substitution, including conservative and non-conservative substitution, or insertion, and wherein said alterations may occur at the amino- or carboxy-terminal positions of the reference polypeptide sequence or anywhere between those terminal positions, interspersed either individually among the amino acids in the reference sequence or in one or more contiguous groups within the reference sequence. The number of amino acid alterations for a given % identity is determined by multiplying the total number of amino acids in SEQ ID NO:2 by the integer defining the percent identity divided by 100 and then subtracting that product from said total number of amino acids in SEQ ID NO:2, or:

$$n_a \leq x_a - (x_a \bullet y),$$

wherein $\mathbf{n_a}$ is the number of amino acid alterations, $\mathbf{x_a}$ is the total number of amino acids in SEQ ID NO:2, **y** is, for instance 0.70 for 70%, 0.80 for 80%, 0.85 for 85% etc., and • is the symbol for the multiplication operator, and wherein any non-integer product of $\mathbf{x_a}$ and **y** is rounded down to the nearest integer prior to subtracting it from $\mathbf{x_a}$.

"Isolated" means altered "by the hand of man" from its natural state, *i.e.,* if it occurs in nature, it has been changed or removed from its original environment, or both. For example, a polynucleotide or a polypeptide naturally present in a living organism is not "isolated," but the same polynucleotide or polypeptide separated from the coexisting materials of its natural state is "isolated", as the term is employed herein. Moreover, a polynucleotide or polypeptide that is introduced into an organism by transformation, genetic manipulation or by any other recombinant method is "isolated" even if it is still present in said organism, which organism may be living or non-living.

"Polynucleotide(s)" generally refers to any polyribonucleotide or polydeoxribonucleotide, which may be unmodified RNA or DNA or modified RNA or DNA. "Polynucleotide(s)" include, without limitation, single- and double-stranded DNA, DNA that is a mixture of single- and double-stranded regions or single-, double- and triple-stranded regions, single- and double-stranded RNA, and RNA that is mixture of single- and double-stranded regions, hybrid molecules comprising DNA and RNA that may be single-stranded or, more typically, double-stranded, or triple-stranded regions, or a mixture of single- and double-stranded regions. In addition, "polynucleotide" as used herein refers to triple-stranded regions comprising RNA or DNA or both RNA and DNA. The strands in such regions may be from the same molecule or from different molecules. The regions may include all of one or more of the molecules, but more typically involve only a region of some of the molecules. One of the molecules of a triple-helical region often is an oligonucleotide. As used herein, the term "polynucleotide(s)" also includes DNAs or RNAs as described above that contain one or more modified bases. Thus, DNAs or RNAs with backbones modified for stability or for other reasons are "polynucleotide(s)" as that term is intended herein. Moreover, DNAs or RNAs comprising unusual bases, such as inosine, or modified bases, such as tritylated bases, to name just two examples, are polynucleotides as the term is used herein. It will be appreciated that a great variety of modifications have been made to DNA and RNA that serve many useful purposes known to those of skill in the art. The term "polynucleotide(s)" as it is employed herein embraces such chemically, enzymatically or metabolically modified forms of polynucleotides, as well as the chemical forms of DNA and RNA characteristic of viruses and cells, including, for example, simple and complex cells. "Polynucleotide(s) " also embraces short polynucleotides often referred to as oligonucleotide(s).

"Polypeptide(s)" refers to any peptide or protein comprising two or more amino acids joined to each other by peptide bonds or modified peptide bonds. "Polypeptide(s)" refers to both short chains, commonly referred to as peptides, oligopeptides and oligomers and to longer chains generally referred to as proteins. Polypeptides may contain amino acids other than the 20 gene encoded amino acids. "Polypeptide(s)" include those modified either by natural processes, such as processing and other post-translational modifications, but also by chemical modification techniques. Such modifications are well described in basic texts and in more detailed monographs, as well as in a voluminous research literature, and they are well known to those of skill in the art. It will be appreciated that the same type of modification may be present in the same or varying degree at several sites in a given polypeptide. Also, a given polypeptide may contain many types of modifications. Modifications can occur anywhere in a polypeptide, including the peptide backbone, the amino acid side-chains, and the amino or carboxyl termini. Modifications include, for example, acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cysteine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, glycosylation, lipid attachment, sulfation, gamma-carboxylation of glutamic acid residues, hydroxylation and ADP-ribosylation, selenoylation, sulfation, transfer-RNA mediated addition of amino acids to proteins, such as arginylation, and ubiquitination. See, for instance, *PROTEINS - STRUCTURE AND MOLECULAR PROPERTIES,* 2nd Ed., T. E. Creighton, W. H. Freeman and Company, New York (1993) and Wold, F., Posttranslational Protein Modifications: Perspectives and Prospects, pgs. 1-12 in *POSTTRANSLATIONAL COVALENT MODIFICATION OF PROTEINS,* B. C. Johnson, Ed., Academic Press, New York (1983); Seifter et al., *Meth. Enzymol. 182*:626-646 (1990) and Rattan et al., *Protein Synthesis: Posttranslational Modifications and Aging,* Ann. N.Y. Acad. Sci. 663: 48-62 (1992). Polypeptides may be branched or cyclic, with or without branching. Cyclic, branched and branched circular polypeptides may result from post-translational natural processes and may be made by entirely synthetic methods, as well.

"Variant(s)" as the term is used herein, is a polynucleotide or polypeptide that differs from a reference polynucleotide or polypeptide respectively, but retains essential properties. A typical variant of a polynucleotide differs in nucleotide sequence from another, reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from another, reference polypeptide. Generally, differences are limited so that the sequences of the reference polypeptide and the variant are closely similar overall and, in many regions, identical. A variant and reference polypeptide may differ in amino acid sequence by one or more substitutions, additions, deletions in any combination. A substituted or inserted amino acid residue may or may not be one encoded by the genetic code. A variant of a polynucleotide or polypeptide may be a naturally occurring such as an allelic variant, or it may be a variant that is not known to occur naturally. Non-naturally occurring variants of polynucleotides and polypeptides may be made by mutagenesis techniques, by direct synthesis, and by other recombinant methods known to skilled artisans.

## EXAMPLES

The examples below are carried out using standard techniques, which are well known and routine to those of skill in the art, except where otherwise described in detail. The examples are illustrative, but do not limit the invention.

### Example 1 Strain selection, Library Production and Sequencing

The polynucleotide having a DNA sequence given in Table 1 [SEQ ID NO:1 or 3] was obtained from a library of clones of chromosomal DNA of *Streptococcus pneumoniae* in *E. coli.* The sequencing data from two or more clones containing overlapping *Streptococcus pneumoniae* DNAs was used to construct the contiguous DNA sequence in SEQ ID NO:1. Libraries may be prepared by routine methods, for example:

Methods 1 and 2 below.

Total cellular DNA is isolated from *Streptococcus pneumoniae* 0100993 according to standard procedures and size-fractionated by either of two methods.

### Method 1

Total cellular DNA is mechanically sheared by passage through a needle in order to size-fractionate according to standard procedures. DNA fragments of up to 11kbp in size are rendered blunt by treatment with exonuclease and DNA polymerase, and EcoRI linkers added. Fragments are ligated into the vector Lambda ZapII that has been cut with EcoRI, the library packaged by standard procedures and *E.coli* infected with the packaged library. The library is amplified by standard procedures.

### Method 2

Total cellular DNA is partially hydrolyzed with a one or a combination of restriction enzymes appropriate to generate a series of fragments for cloning into library vectors (e.g., RsaI, PalI, AluI, Bsh1235I), and such fragments are size-fractionated according to standard procedures. EcoRI linkers are ligated to the DNA and the fragments then ligated into the vector Lambda ZapII that have been cut with EcoRI, the library packaged by standard procedures, and *E.coli* infected with the packaged library. The library is amplified by standard procedures.

## Claims

1. An isolated polynucleotide comprising a polynucleotide having at least a 70% identity to a polynucleotide encoding a polypeptide comprising the amino acid sequence of SEQ ID NO:2.

2. An isolated polynucleotide comprising a polynucleotide having at least a 70% identity to a polynucleotide encoding the same mature polypeptide expressed by the SecA gene contained in the *Streptococcus pneumoniae.*

3. An isolated polynucleotide comprising a polynucleotide encoding a polypeptide comprising an amino acid sequence which is at least 70% identical to the amino acid sequence of SEQ ID NO:2.

4. An isolated polynucleotide that is complementary to the polynucleotide of claim 1

5. The polynucleotide of Claim 1 wherein the polynucleotide is DNA or RNA

6. The polynucleotide of Claim 1 comprising the nucleic acid sequence set forth in SEQ ID NO:1.

7. The polynucleotide of Claim 1 comprising nucleotide 1 to the stop codon which begins at nucleotide number 2512 set forth in SEQ ID NO:1.

8. The polynucleotide of Claim 1 which encodes a polypeptide comprising the amino acid sequence of SEQ ID NO:2.

9. A vector comprising the polynucleotide of Claim 1.

10. A host cell comprising the vector of Claim 9.

11. A process for producing a polypeptide comprising: expressing from the host cell of Claim 10 a polypeptide encoded by said DNA.

12. A process for producing a SecA polypeptide or fragment comprising culturing a host of claim 10 under conditions sufficient for the production of said polypeptide or fragment.

13. A polypeptide comprising an amino acid sequence which is at least 70% identical to the amino acid sequence of SEQ ID NO:2.

14. A polypeptide comprising an amino acid sequence as set forth in SEQ ID NO:2.

15. An antibody against the polypeptide of claim 14.

16. An antagonist which inhibits the activity or expression of the polypeptide of claim 14.

17. A method for the treatment of an individual in need of SecA polypeptide comprising: administering to the individual a therapeutically effective amount of the polypeptide of claim 14.

18. A method for the treatment of an individual having need to inhibit SecA polypeptide comprising: administering to the individual a therapeutically effective amount of the antagonist of Claim 18.

19. A process for diagnosing a disease related to expression or activity of the polypeptide of claim 14 in an individual comprising:

    (a) determining a nucleic acid sequence encoding said polypeptide, and/or
    (b) analyzing for the presence or amount of said polypeptide in a sample derived from the individual.

20. A method for identifying compounds which interact with and inhibit or activate an activity of the polypeptide of claim 14 comprising:

    contacting a composition comprising the polypeptide with the compound to be screened under conditions to permit interaction between the compound and the polypeptide to assess the interaction of a compound, such interaction being associated with a second component capable of providing a detectable signal in response to the interaction of the polypeptide with the compound;
    and determining whether the compound interacts with and activates or inhibits an activity of the polypeptide by detecting the presence or absence of a signal generated from the interaction of the compound with the polypeptide.

**21.** A method for inducing an immunological response in a mammal which comprises inoculating the mammal with SecA polypeptide of claim 14, or a fragment or variant thereof, adequate to produce antibody and/or T cell immune response to protect said animal from disease.

**22.** A method of inducing immunological response in a mammal which comprises delivering a nucleic acid vector to direct expression of SecA polypeptide of claim 14, or fragment or a variant thereof, for expressing said SecA polypeptide, or a fragment or a variant thereof *in vivo* in order to induce an immunological response to produce antibody and/ or T cell immune response to protect said animal from disease.

**23.** A computer readable medium having stored thereon a member selected from the group consisting of: a polynucleotide comprising the sequence of SEQ ID NO. 1 or 3; a polypeptide comprising the sequence of SEQ ID NO. 2 or 4; a set of polynucleotide sequences wherein at least one of said sequences comprises the sequence of SEQ ID NO. 1 or 3; a set of polypeptide sequences wherein at least one of said sequences comprises the sequence of SEQ ID NO. 2 or 4; a data set representing a polynucleotide sequence comprising the sequence of SEQ ID NO. 1 or 3; a data set representing a polynucleotide sequence encoding a polypeptide sequence comprising the sequence of SEQ ID NO. 2 or 4; a polynucleotide comprising the sequence of SEQ ID NO. 1; a polypeptide comprising the sequence of SEQ ID NO. 2; a set of polynucleotide sequences wherein at least one of said sequences comprises the sequence of SEQ ID NO. 1; a set of polypeptide sequences wherein at least one of said sequences comprises the sequence of SEQ ID NO. 2; a data set representing a polynucleotide sequence comprising the sequence of SEQ ID NO. 1; a data set representing a polynucleotide sequence encoding a polypeptide sequence comprising the sequence of SEQ ID NO. 2.

**24.** A computer based method for performing homology identification, said method comprising the steps of providing a polynucleotide sequence comprising the sequence of SEQ ID NO. 1 in a computer readable medium; and comparing said polynucleotide sequence to at least one polynucleotide or polypeptide sequence to identify homology.

**25.** A further embodiment of the invention provides a computer based method for polynucleotide assembly, said method comprising the steps of: providing a first polynucleotide sequence comprising the sequence of SEQ ID NO. 1 in a computer readable medium; and screening for at least one overlapping region between said first polynucleotide sequence and a second polynucleotide sequence.

**26.** An isolated polynucleotide comprising a polynucleotide having at least a 70% identity to a polynucleotide encoding a polypeptide comprising the amino acid sequence of SEQ ID NO:4.

**27.** An isolated polynucleotide comprising a polynucleotide having at least a 70% identity to the polynucleotide sequence of SEQ ID NO:3.

**Annex to the description**

SEQUENCE LISTING

(1) GENERAL INFORMATION:

(i) APPLICANT: SmithKline Beecham Corporation & SmithKline Beecham Plc

(ii) TITLE OF INVENTION: SecA

(iii) NUMBER OF SEQUENCES: 6

(iv) CORRESPONDENCE ADDRESS:
(A) ADDRESSEE: SmithKline Beecham, Corporate Intellectual Property
(B) STREET: Two New Horizons Court
(C) CITY: Brentford
(D) STATE: Middlesex
(E) COUNTRY: United Kingdom
(F) ZIP: TW8 9EP

(v) COMPUTER READABLE FORM:
(A) MEDIUM TYPE: Diskette
(B) COMPUTER: IBM Compatible
(C) OPERATING SYSTEM: Windows 95
(D) SOFTWARE: FastSEQ for Windows Version 2.0b

(vi) CURRENT APPLICATION DATA:
(A) APPLICATION NUMBER:
(B) FILING DATE:
(C) CLASSIFICATION:

(vii) PRIOR APPLICATION DATA:
(A) APPLICATION NUMBER: 60/054,568
(B) FILING DATE: 01-AUG-1997

(viii) ATTORNEY/AGENT INFORMATION:
(A) NAME: CONNELL, Anthony Christopher
(B) REGISTRATION NUMBER: 5630 & 26758
(C) REFERENCE/DOCKET NUMBER: GM10061

(ix) TELECOMMUNICATION INFORMATION:
(A) TELEPHONE: +44 1279 644 395
(B) TELEFAX: +44 181 975 6294
(C) TELEX:

(2) INFORMATION FOR SEQ ID NO:1:

(i) SEQUENCE CHARACTERISTICS:
(A) LENGTH: 2511 base pairs
(B) TYPE: nucleic acid
(C) STRANDEDNESS: double
(D) TOPOLOGY: linear

(xi) SEQUENCE DESCRIPTION: SEQ ID NO:1:

```
ATGGCTAATA TTTTAAAAAC AATTATCGAA AATGATAAAG GAGAAATCCG TCGTCTGGAA     60
AAGATGGCTG ACAAGGTTTT CAAATACGAA GACCAAATGG CTGCTTTGAC TGACGACCAA    120
CTAAAAGCAA AAACAGTTGA ATTTAAGGAA CGTTATCAAA ATGGAGAATC ACTGGATTCA    180
TTGCTTTACG AAGCATTTGC GGTTGTCCGT GAAGGTGCCA AACGTGTCCT AGGTCTCTTC    240
CCTTATAAGG TTCAGGTCAT GGGGGGGATT GTTCTTCACC ATGGTGACGT GCCAGAGATG    300
CGTACAGGGG AAGGGAAAAC CTTGACTGCG ACCATGCCGG TATACCTCAA TGCCCTTTCA    360
GGTAAAGGGG TTCACGTAGT TACGGTTAAT GAATACCTGT CAGAACGTGA CGCGACTGAG    420
ATGGGTGAAT TGTACTCTTG GCTTGGTTTG TCAGTAGGGA TTAACTTGGC TACCAAATCT    480
CCAATGGAGA AAAAAGAAGC CTATGAGTGT GATATTACTT ACTCAACTAA CTCAGAAATC    540
GGATTTGACT ACCTTCGTGA CAATATGGTC GTTCGCGCTG AAAACATGGT ACAACGTCCG    600
CTTAACTATG CCTTGGTCGA TGAGGTTGAC TCTATCTTGA TTGACGAGGC TCGTACACCT    660
TTGATTGTAT CAGGTGCCAA TGCGGTTGAA ACCAGTCAGT TGTATCACAT GGCAGACCAC    720
TATGTAAAAT CTTTGAACAA AGATGACTAC ATCATCGATG TGCAGTCTAA GACTATTGGT    780
TTGTCTGATT CAGGGATTGA CAGGGCTGAA AGCTACTTCA AACTTGAAAA CCTCTATGAC    840
ATCGAAAACG TGGCTTTGAC CCACTTTATC GATAACGCCC TTCGTGCCAA CTACATCATG    900
CTTCTCGATA TTGACTATGT GGTGAGCGAA GAGCAAGAAA TCTTGATTGT CGACCAATTT    960
ACAGGTCGTA CCATGGAAGG TCGTCGTTAT TCTGATGGAT TGCACCAAGC TATTGAAGCC   1020
AAAGAAGGTG TGCCAATCCA GGATGAAACC AAGACATCTG CCTCAATCAC GTACCAAAAC   1080
```

```
CTTTTCCGTA TGTACAAAAA ATTGTCTGGT ATGACGGGTA CAGGTAAGAC TGAGGAAGAA   1140
GAATTTCGTG AAATCTACAA CATTCGTGTT ATTCCAATCC CAACAAACCG TCCTGTTCAA   1200
CGTATTGACC ACTCAGACCT TCTTTATGCA AGTATCGAAT CTAAGTTTAA AGCGGTTGTC   1260
GAAGACGTTA AGGCTCGTTA CCAAAAGGGT CAACCTGTCT TGGTTGGTAC AGTAGCGGTT   1320
GAAACTAGTG ACTACATTTC TAAGAAATTG GTTGCAGCTG GTGTTCCTCA CGAAGTCTTG   1380
AATGCCAAAA ACCACTATAG AGAAGCCCAA ATCATCATGA ATGCTGGTCA ACGTGGTGCC   1440
GTTACCATCG CAACCAACAT GGCGGGTCGT GGTACCGACA TCAAGCTTGG TGAAGGTGTT   1500
CGTGAACTTG GAGGACTTTG TGTTATTGGT ACAGAACGTC ATGAAAGTCG TCGTATCGAT   1560
AACCAGCTTC GTGGACGTTC AGGTCGTCAA GGAGATCCAG GTGAGTCACA ATTCTACCTA   1620
TCTCTTGAAG ATGATTTGAT GAAACGTTTT GGTTCTGAAC GCTTGAAGGG AATCTTTGAA   1680
CGCTTGAACA TGTCTGAAGA GGCCATTGAG TCTCGCATGT TGACGCGTCA GGTTGAAGCA   1740
GCTCAGAAAC GTGTCGAAGG AAATAACTAC GATACCCGTA AACAAGTCCT TCAATACGAT   1800
GATGTCATGC GTGAACAACG TGAGATTATC TATGCTCAAC GTTACGATGT CATCACTGCA   1860
GATCGTGACT TGGCACCTGA AATTCAGTCT ATGATTAAGC GCACGATTGA ACGTGTCGTT   1920
GATGGTCATG CGCGTGCCAA ACAAGATGAA AAACTAGAGG CAATTTTGAA CTTTGCTAAG   1980
TACAACTTGC TTCCTGAAGA TTCTATTACG ATGGAAGACT TGTCAGGCTT GTCTGATAAG   2040
GCCATCAAGG AAGAGCTTTT CCAACGTGCC TTGAAGGTTT ACGATAGTCA GGTTTCAAAA   2100
CTACGCGATG AAGAAGCAGT TAAAGAATTC CAAAAAGTTT TGATTCTACG AGTGGTGGAT   2160
AACAAGTGGA CAGATCATAT CGATGCCCTT GATCAATTGC GTAACGCGGT TGGACTTCGT   2220
GGCTATGCTC AGAACAACCC TGTTGTTGAG TATCAGGCAG AAGGTTTCCG TATGTTTAAT   2280
GATATGATTG GTTCGATTGA GTTTGATGTG ACACGCTTGA TGATGAAAGC ACAAATTCAT   2340
GAACAAGAAA GACCACAGGC AGAACGTCAT ATCAGTACAA CAGCGACTCG CAATATCGCT   2400
GCTCACCAAG CAAGTATGCT AGAAGATTTG GATTTGAGCC AGATTGGACG CAATGAACTT   2460
TGCCCATGTG GTTCTGGTAA GAAATTTAAA AACTGTCACG GTAAAAGACA A            2511
```

(2) INFORMATION FOR SEQ ID NO:2:

(i) SEQUENCE CHARACTERISTICS:

(A) LENGTH: 837 amino acids

(B) TYPE: amino acid

(C) STRANDEDNESS: single

(D) TOPOLOGY: linear

(xi) SEQUENCE DESCRIPTION: SEQ ID NO:2:

```
Met Ala Asn Ile Leu Lys Thr Ile Ile Glu Asn Asp Lys Gly Glu Ile
 1               5                  10                  15
Arg Arg Leu Glu Lys Met Ala Asp Lys Val Phe Lys Tyr Glu Asp Gln
            20                  25                  30
```

Met Ala Ala Leu Thr Asp Asp Gln Leu Lys Ala Lys Thr Val Glu Phe
35 40 45
Lys Glu Arg Tyr Gln Asn Gly Glu Ser Leu Asp Ser Leu Leu Tyr Glu
50 55 60
Ala Phe Ala Val Val Arg Glu Gly Ala Lys Arg Val Leu Gly Leu Phe
65 70 75 80
Pro Tyr Lys Val Gln Val Met Gly Gly Ile Val Leu His His Gly Asp
85 90 95
Val Pro Glu Met Arg Thr Gly Glu Gly Lys Thr Leu Thr Ala Thr Met
100 105 110
Pro Val Tyr Leu Asn Ala Leu Ser Gly Lys Gly Val His Val Val Thr
115 120 125
Val Asn Glu Tyr Leu Ser Glu Arg Asp Ala Thr Glu Met Gly Glu Leu
130 135 140
Tyr Ser Trp Leu Gly Leu Ser Val Gly Ile Asn Leu Ala Thr Lys Ser
145 150 155 160
Pro Met Glu Lys Lys Glu Ala Tyr Glu Cys Asp Ile Thr Tyr Ser Thr
165 170 175
Asn Ser Glu Ile Gly Phe Asp Tyr Leu Arg Asp Asn Met Val Val Arg
180 185 190
Ala Glu Asn Met Val Gln Arg Pro Leu Asn Tyr Ala Leu Val Asp Glu
195 200 205
Val Asp Ser Ile Leu Ile Asp Glu Ala Arg Thr Pro Leu Ile Val Ser
210 215 220
Gly Ala Asn Ala Val Glu Thr Ser Gln Leu Tyr His Met Ala Asp His
225 230 235 240
Tyr Val Lys Ser Leu Asn Lys Asp Asp Tyr Ile Ile Asp Val Gln Ser
245 250 255
Lys Thr Ile Gly Leu Ser Asp Ser Gly Ile Asp Arg Ala Glu Ser Tyr
260 265 270
Phe Lys Leu Glu Asn Leu Tyr Asp Ile Glu Asn Val Ala Leu Thr His
275 280 285
Phe Ile Asp Asn Ala Leu Arg Ala Asn Tyr Ile Met Leu Leu Asp Ile
290 295 300
Asp Tyr Val Val Ser Glu Glu Gln Glu Ile Leu Ile Val Asp Gln Phe
305 310 315 320
Thr Gly Arg Thr Met Glu Gly Arg Arg Tyr Ser Asp Gly Leu His Gln
325 330 335
Ala Ile Glu Ala Lys Glu Gly Val Pro Ile Gln Asp Glu Thr Lys Thr
340 345 350

```
Ser Ala Ser Ile Thr Tyr Gln Asn Leu Phe Arg Met Tyr Lys Lys Leu
        355                 360                 365
Ser Gly Met Thr Gly Thr Gly Lys Thr Glu Glu Glu Glu Phe Arg Glu
    370                 375                 380
Ile Tyr Asn Ile Arg Val Ile Pro Ile Pro Thr Asn Arg Pro Val Gln
385                 390                 395                 400
Arg Ile Asp His Ser Asp Leu Leu Tyr Ala Ser Ile Glu Ser Lys Phe
                405                 410                 415
Lys Ala Val Val Glu Asp Val Lys Ala Arg Tyr Gln Lys Gly Gln Pro
            420                 425                 430
Val Leu Val Gly Thr Val Ala Val Glu Thr Ser Asp Tyr Ile Ser Lys
        435                 440                 445
Lys Leu Val Ala Ala Gly Val Pro His Glu Val Leu Asn Ala Lys Asn
    450                 455                 460
His Tyr Arg Glu Ala Gln Ile Ile Met Asn Ala Gly Gln Arg Gly Ala
465                 470                 475                 480
Val Thr Ile Ala Thr Asn Met Ala Gly Arg Gly Thr Asp Ile Lys Leu
                485                 490                 495
Gly Glu Gly Val Arg Glu Leu Gly Gly Leu Cys Val Ile Gly Thr Glu
            500                 505                 510
Arg His Glu Ser Arg Arg Ile Asp Asn Gln Leu Arg Gly Arg Ser Gly
        515                 520                 525
Arg Gln Gly Asp Pro Gly Glu Ser Gln Phe Tyr Leu Ser Leu Glu Asp
    530                 535                 540
Asp Leu Met Lys Arg Phe Gly Ser Glu Arg Leu Lys Gly Ile Phe Glu
545                 550                 555                 560
Arg Leu Asn Met Ser Glu Glu Ala Ile Glu Ser Arg Met Leu Thr Arg
                565                 570                 575
Gln Val Glu Ala Ala Gln Lys Arg Val Glu Gly Asn Asn Tyr Asp Thr
            580                 585                 590
Arg Lys Gln Val Leu Gln Tyr Asp Asp Val Met Arg Glu Gln Arg Glu
        595                 600                 605
Ile Ile Tyr Ala Gln Arg Tyr Asp Val Ile Thr Ala Asp Arg Asp Leu
    610                 615                 620
Ala Pro Glu Ile Gln Ser Met Ile Lys Arg Thr Ile Glu Arg Val Val
625                 630                 635                 640
Asp Gly His Ala Arg Ala Lys Gln Asp Glu Lys Leu Glu Ala Ile Leu
                645                 650                 655
Asn Phe Ala Lys Tyr Asn Leu Leu Pro Glu Asp Ser Ile Thr Met Glu
            660                 665                 670
```

Asp Leu Ser Gly Leu Ser Asp Lys Ala Ile Lys Glu Glu Leu Phe Gln
675 680 685

Arg Ala Leu Lys Val Tyr Asp Ser Gln Val Ser Lys Leu Arg Asp Glu
690 695 700

Glu Ala Val Lys Glu Phe Gln Lys Val Leu Ile Leu Arg Val Val Asp
705 710 715 720

Asn Lys Trp Thr Asp His Ile Asp Ala Leu Asp Gln Leu Arg Asn Ala
725 730 735

Val Gly Leu Arg Gly Tyr Ala Gln Asn Asn Pro Val Val Glu Tyr Gln
740 745 750

Ala Glu Gly Phe Arg Met Phe Asn Asp Met Ile Gly Ser Ile Glu Phe
755 760 765

Asp Val Thr Arg Leu Met Met Lys Ala Gln Ile His Glu Gln Glu Arg
770 775 780

Pro Gln Ala Glu Arg His Ile Ser Thr Thr Ala Thr Arg Asn Ile Ala
785 790 795 800

Ala His Gln Ala Ser Met Leu Glu Asp Leu Asp Leu Ser Gln Ile Gly
805 810 815

Arg Asn Glu Leu Cys Pro Cys Gly Ser Gly Lys Lys Phe Lys Asn Cys
820 825 830

His Gly Lys Arg Gln
835

(2) INFORMATION FOR SEQ ID NO:3:

(i) SEQUENCE CHARACTERISTICS:
(A) LENGTH: 2079 base pairs
(B) TYPE: nucleic acid
(C) STRANDEDNESS: double
(D) TOPOLOGY: linear

(xi) SEQUENCE DESCRIPTION: SEQ ID NO:3:

TACTCTTGGC TTGGTTTGTC AGTAGGGATT AACTTGGCTA CCAAATCTCC AATGGAGAAA 60
AAAGAAGCCT ATGAGTGTGA TATTACTTAC TCAACTAACT CAGAAATCGG ATTTGACTAC 120
CTTCGTGACA ATATGGTCGT TCGCGCTGAA AACATGGTAC AACGTCCGCT TAACTATGCC 180
TTGGTCGATG AGGTTGACTC TATCTTGATT GACGAGGCTC GTACACCTTT GATTGTATCA 240
GGTGCCAATG CGGTTGAAAC CAGTCAGTTG TATCACATGG CAGACCACTA TGTAAAATCT 300
TTGAACAAAG ATGACTACAT CATCGATGTG CAGTCTAAGA CTATTGGTTT GTCTGATTCA 360

```
GGGATTGACA GGGCTGAAAG CTACTTCAAA CTTGAAAACC TCTATGACAT CGAAAACGTG    420
GCTTTGACCC ACTTTATCGA TAACGCCCTT CGTGCCAACT ACATCATGCT TCTCGATATT    480
GACTATGTGG TGAGCGAAGA GCAAGAAATC TTGATTGTCG ACCAATTTAC AGGTCGTACC    540
ATGGAAGGTC GTCGTTATTC TGATGGATTG CACCAAGCTA TTGAAGCCAA AGAAGGTGTG    600
CCAATCCAGG ATGAAACCAA GACATCTGCC TCAATCACGT ACCAAAACCT TTTCCGTATG    660
TACAAAAAAT TGTCTGGTAT GACGGGTACA GGTAAGACTG AGGAAGAAGA ATTTCGTGAA    720
ATCTACAACA TTCGTGTTAT TCCAATCCCA ACAAACCGTC CTGTTCAACG TATTGACCAC    780
TCAGACCTTC TTTATGCAAG TATCGAATCT AAGTTTAAAG CGGTTGTCGA AGACGTTAAG    840
GCTCGTTACC AAAAGGGTCA ACCTGTCTTG GTTGGTACAG TAGCGGTTGA AACTAGTGAC    900
TACATTTCTA AGAAATTGGT TGCAGCTGGT GTTCCTCACG AAGTCTTGAA TGCCAAAAAC    960
CACTATAGAG AAGCCCAAAT CATCATGAAT GCTGGTCAAC GTGGTGCCGT TACCATCGCA   1020
ACCAACATGG CGGGTCGTGG TACCGACATC AAGCTTGGTG AAGGTGTTCG TGAACTTGGA   1080
GGACTTTGTG TTATTGGTAC AGAACGTCAT GAAAGTCGTC GTATCGATAA CCAGCTTCGT   1140
GGACGTTCAG GTCGTCAAGG AGATCCAGGT GAGTCACAAT TCTACCTATC TCTTGAAGAT   1200
GATTTGATGA AACGTTTTGG TTCTGAACGC TTGAAGGGAA TCTTTGAACG CTTGAACATG   1260
TCTGAAGAGG CCATTGAGTC TCGCATGTTG ACGCGTCAGG TTGAAGCAGC TCAGAAACGT   1320
GTCGAAGGAA ATAACTACGA TACCCGTAAA CAAGTCCTTC AATACGATGA TGTCATGCGT   1380
GAACAACGTG AGATTATCTA TGCTCAACGT TACGATGTCA TCACTGCAGA TCGTGACTTG   1440
GCACCTGAAA TTCAGTCTAT GATTAAGCGC ACGATTGAAC GTGTCGTTGA TGGTCATGCG   1500
CGTGCCAAAC AAGATGAAAA ACTAGAGGCA ATTTTGAACT TTGCTAAGTA CAACTTGCTT   1560
CCTGAAGATT CTATTACGAT GGAAGACTTG TCAGGCTTGT CTGATAAGGC CATCAAGGAA   1620
GAGCTTTTCC AACGTGCCTT GAAGGTTTAC GATAGTCAGG TTTCAAAACT ACGCCGATGAA  1680
GAAGCAGTTA AAGAATTCCA AAAAGTTTTG ATTCTACGAG TGGTGGATAA CAAGTGGACA   1740
GATCATATCG ATGCCCTTGA TCAATTGCGT AACGCGGTTG GACTTCGTGG CTATGCTCAG   1800
AACAACCCTG TTGTTGAGTA TCAGGCAGAA GGTTTCCGTA TGTTTAATGA TATGATTGGT   1860
TCGATTGAGT TTGATGTGAC ACGCTTGATG ATGAAAGCAC AAATTCATGA ACAAGAAAGA   1920
CCACAGGCAG AACGTCATAT CAGTACAACA GCGACTCGCA ATATCGCTGC TCACCAAGCA   1980
AGTATGCTAG AAGATTTGGA TTTGAGCCAG ATTGGACGCA ATGAACTTTG CCCATGTGGT   2040
TCTGGTAAGA AATTTAAAAA CTGTCACGGT AAAAGACAA                          2079
```

(2) INFORMATION FOR SEQ ID NO:4:

(i) SEQUENCE CHARACTERISTICS:

(A) LENGTH: 693 amino acids

(B) TYPE: amino acid

(C) STRANDEDNESS: single

(D) TOPOLOGY: linear

(xi) SEQUENCE DESCRIPTION: SEQ ID NO:4:

```
Tyr Ser Trp Leu Gly Leu Ser Val Gly Ile Asn Leu Ala Thr Lys Ser
 1               5                  10                  15
Pro Met Glu Lys Lys Glu Ala Tyr Glu Cys Asp Ile Thr Tyr Ser Thr
            20                  25                  30
Asn Ser Glu Ile Gly Phe Asp Tyr Leu Arg Asp Asn Met Val Val Arg
        35                  40                  45
Ala Glu Asn Met Val Gln Arg Pro Leu Asn Tyr Ala Leu Val Asp Glu
    50                  55                  60
Val Asp Ser Ile Leu Ile Asp Glu Ala Arg Thr Pro Leu Ile Val Ser
65                  70                  75                  80
Gly Ala Asn Ala Val Glu Thr Ser Gln Leu Tyr His Met Ala Asp His
                85                  90                  95
Tyr Val Lys Ser Leu Asn Lys Asp Asp Tyr Ile Ile Asp Val Gln Ser
            100                 105                 110
Lys Thr Ile Gly Leu Ser Asp Ser Gly Ile Asp Arg Ala Glu Ser Tyr
        115                 120                 125
Phe Lys Leu Glu Asn Leu Tyr Asp Ile Glu Asn Val Ala Leu Thr His
    130                 135                 140
Phe Ile Asp Asn Ala Leu Arg Ala Asn Tyr Ile Met Leu Leu Asp Ile
145                 150                 155                 160
Asp Tyr Val Val Ser Glu Glu Gln Glu Ile Leu Ile Val Asp Gln Phe
                165                 170                 175
Thr Gly Arg Thr Met Glu Gly Arg Arg Tyr Ser Asp Gly Leu His Gln
            180                 185                 190
Ala Ile Glu Ala Lys Glu Gly Val Pro Ile Gln Asp Glu Thr Lys Thr
        195                 200                 205
Ser Ala Ser Ile Thr Tyr Gln Asn Leu Phe Arg Met Tyr Lys Lys Leu
    210                 215                 220
Ser Gly Met Thr Gly Thr Gly Lys Thr Glu Glu Glu Glu Phe Arg Glu
225                 230                 235                 240
Ile Tyr Asn Ile Arg Val Ile Pro Ile Pro Thr Asn Arg Pro Val Gln
                245                 250                 255
Arg Ile Asp His Ser Asp Leu Leu Tyr Ala Ser Ile Glu Ser Lys Phe
            260                 265                 270
Lys Ala Val Val Glu Asp Val Lys Ala Arg Tyr Gln Lys Gly Gln Pro
        275                 280                 285
Val Leu Val Gly Thr Val Ala Val Glu Thr Ser Asp Tyr Ile Ser Lys
    290                 295                 300
Lys Leu Val Ala Ala Gly Val Pro His Glu Val Leu Asn Ala Lys Asn
```

```
305                 310                 315                 320
His Tyr Arg Glu Ala Gln Ile Ile Met Asn Ala Gly Gln Arg Gly Ala
                325                 330                 335
Val Thr Ile Ala Thr Asn Met Ala Gly Arg Gly Thr Asp Ile Lys Leu
            340                 345                 350
Gly Glu Gly Val Arg Glu Leu Gly Gly Leu Cys Val Ile Gly Thr Glu
        355                 360                 365
Arg His Glu Ser Arg Arg Ile Asp Asn Gln Leu Arg Gly Arg Ser Gly
    370                 375                 380
Arg Gln Gly Asp Pro Gly Glu Ser Gln Phe Tyr Leu Ser Leu Glu Asp
385                 390                 395                 400
Asp Leu Met Lys Arg Phe Gly Ser Glu Arg Leu Lys Gly Ile Phe Glu
                405                 410                 415
Arg Leu Asn Met Ser Glu Glu Ala Ile Glu Ser Arg Met Leu Thr Arg
            420                 425                 430
Gln Val Glu Ala Ala Gln Lys Arg Val Glu Gly Asn Asn Tyr Asp Thr
        435                 440                 445
Arg Lys Gln Val Leu Gln Tyr Asp Asp Val Met Arg Glu Gln Arg Glu
    450                 455                 460
Ile Ile Tyr Ala Gln Arg Tyr Asp Val Ile Thr Ala Asp Arg Asp Leu
465                 470                 475                 480
Ala Pro Glu Ile Gln Ser Met Ile Lys Arg Thr Ile Glu Arg Val Val
                485                 490                 495
Asp Gly His Ala Arg Ala Lys Gln Asp Glu Lys Leu Glu Ala Ile Leu
            500                 505                 510
Asn Phe Ala Lys Tyr Asn Leu Leu Pro Glu Asp Ser Ile Thr Met Glu
        515                 520                 525
Asp Leu Ser Gly Leu Ser Asp Lys Ala Ile Lys Glu Glu Leu Phe Gln
    530                 535                 540
Arg Ala Leu Lys Val Tyr Asp Ser Gln Val Ser Lys Leu Arg Asp Glu
545                 550                 555                 560
Glu Ala Val Lys Glu Phe Gln Lys Val Leu Ile Leu Arg Val Val Asp
                565                 570                 575
Asn Lys Trp Thr Asp His Ile Asp Ala Leu Asp Gln Leu Arg Asn Ala
            580                 585                 590
Val Gly Leu Arg Gly Tyr Ala Gln Asn Asn Pro Val Val Glu Tyr Gln
        595                 600                 605
Ala Glu Gly Phe Arg Met Phe Asn Asp Met Ile Gly Ser Ile Glu Phe
    610                 615                 620
Asp Val Thr Arg Leu Met Met Lys Ala Gln Ile His Glu Gln Glu Arg
```

```
625                 630                 635                 640
Pro Gln Ala Glu Arg His Ile Ser Thr Thr Ala Thr Arg Asn Ile Ala
                645                 650                 655
Ala His Gln Ala Ser Met Leu Glu Asp Leu Asp Leu Ser Gln Ile Gly
            660                 665                 670
Arg Asn Glu Leu Cys Pro Cys Gly Ser Gly Lys Lys Phe Lys Asn Cys
        675                 680                 685
His Gly Lys Arg Gln
    690
```

(2) INFORMATION FOR SEQ ID NO:5:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO:5:

ATGGCTAATA TTTTAAAAAC 20

(2) INFORMATION FOR SEQ ID NO:6:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO:6:

TTATTGTCTT TTACCGTGAC 20